# EUROPEAN PATENT SPECIFICATION

(11) **EP 1 289 431 B1**
(45) Date of publication and mention of the grant of the patent: **14.01.2009**
(21) Application number: 01939195.2
(22) Date of filing: 17.05.2001
(51) Int. Cl.: A61B 17/08, A61B 19/00

(54) **TISSUE APPROXIMATION DEVICE**
ANGLEICHVORRICHTUNG FÜR GEWEBE
DISPOSITIF DE DISTRIBUTION DE TENSION DE TISSU MULTIPOINTS, RESTAURATION DU CADRE ORBITAIRE ET VARIATION DE SUSPENSION COMBINEES

(30) Priority: 19.05.2000 US 574603; 16.02.2001 US 788118; 22.03.2001 US 816641
(43) Date of publication of application: 12.03.2003
(73) Proprietor: Coapt Systems, Inc., Palo Alto, CA 94303 (US)
(72) Inventor: JACOBS, Daniel, Palo Alto, CA 94306 (US); ELSON, Robert, James, Los Altos, CA 94022 (US); PAGANELLI, Jude, V., Kentfield, CA 94904 (US)
(74) Representative: Price, Nigel John King
(86) International application number: PCT/US2001/016346
(87) International publication number: WO 2001/089392

(56) References cited:
- WO-A-00/49983
- FR-A- 2 744 623
- US-A- 4 430 998
- US-A- 5 047 047
- US-A- 5 425 747
- US-A- 5 919 234

## Description

### FIELD OF THE INVENTION

This invention is in the field of surgery. More particularly, it relates to tissue approximation devices. By "approximation" we mean to include variously the specific movement of two regions of tissue towards each other, the movement of one or more selected tissue regions or areas, the maintenance and/or fixation of one or more selected tissue regions in a selected position, and the maintenance and/or fixation of a selected area of tissue against shape variation due to tissue "springiness." We will also refer to these functions as "stabilization" of a tissue region. For instance, the inventive device may be used to facilitate wound healing by holding soft tissue together under improved distribution of tension and with minimal disruption of the wound interface and its nutrient supplies. Generally, the device has multiple sites for grasping said tissue using tines or prongs or other generally sharp, projecting points, extending from and preferably affixed to a single, supportive backing.

### BACKGROUND OF THE INVENTION

The inventive device is preferably used for the approximation, mobilization, or fixation of tissue. As noted above, these terms are meant variously to include the specific movement of two regions of tissue towards each other, the movement of one or more selected tissue regions or areas, the maintenance of one or more selected tissue regions in a selected position, and the maintenance of a selected area of tissue against shape variation due to tissue "springiness." Using our inventive device, a variety of approximation procedures may be achieved, variously from the movement of two tissue areas towards each other at a common wound margin to the maintenance of an area of tissue in a specific position during or after a surgical procedure, e.g. brow lifts or ACL regions.

For instance, our inventive device allows healing of soft tissue due to its maintenance of tissue position. The surgically induced healing of soft tissue wounds involves two phases, the mechanical phase of wound closure followed by the biochemical phase which involves protein bridging and scanning. In the mechanical phase, the edges of soft tissue are held in contact by essentially two components: 1) The physical properties and device-tissue interactions of the materials holding the tissue edges in contact, e.g. sutures or staples; and 2) An early deposition of proteinaceous material that has adhesive characteristics, e.g. fibrin glue.

Only in the biochemical phase, which occurs after the mechanical phase, do tissue components replace the mechanical components adhering the wound surfaces. During the biochemical phase, the inflammatory cascade generates signals which induce fibroblasts to migrate into the wound and synthesize collagen fibers.

Collagen is the primary constituent of connective tissue and ultimately determines the pliability and tensile strength of the healing wound. Tensile strength is gradually recovered; 60% of ultimate wound strength is achieved after approximately 3 months. However, this process is successful only if the previous mechanical phase has proceeded normally.

The surgeon's goal is to optimize the strength and often the cosmetic appearance of a wound closure or tissue coaptation. For this to happen, tissue is mechanically approximated until the wound has healed enough to withstand stress without artificial support. Optimal healing requires the application of appropriate tissue tension on the closure to eliminate dead space but not create ischemia within the tissue. Both of these circumstances increase the risk of wound infection and wound dehiscence.

Although the biomaterial composition of sutures has progressed considerably, the sophistication of manual suture placement in wounds has advanced relatively little since the original use of fabrics several thousand years ago to tie wound edges together. The wide tolerance ranges for suture placement, tension, and configurations, both amongst different surgeons and for different implementations by the same surgeon, result in a significant component of sub-optimal technique. Yet, the technique used for wound closure forms the foundation for all subsequent events in the healing process. It is during this mechanical phase that tissue tension is high, edema and inflammation are intense, wound edge ischemia is greatest, and that one can already observe the complication of wound failure.

Soft tissue is well known for its inability to hold tension. Even when optimally placed, sutures gradually tear through soft tissue, producing gaps in wounds and possibly leading to the eventual failure or sub-optimization of wound healing. Furthermore, since sutures require the implementation of high levels of tension to counteract the forces acting to separate tissues, they may strangulate the blood supply of the tissues through which they are placed, thus inhibiting the delivery of wound nutrients and oxygen necessary for healing.

There have been many attempts to construct wound closure devices that decrease closure time and improve cosmesis. U.S. Pat. Nos. 2,421,193 and 2,472,009 to Gardner; 4,430,998 to Harvey et al.; 4,535,772 to Sheehan; 4,865,026 to Barrett; 5,179,964 to Cook; and 5,531,760 to Alwafaie suggest such devices. However, these devices are not useful in surgical or deeper wounds. They only approximate the skin surface, joining skin edges variously through external approaches, using adhesives or nonabsorbable attachment points that penetrate tissue. The devices minimally improve the biomechanics of wound closure, and do not adequately approximate the deeper layers of the closure, i.e. fascia or dermis. Externally placed attachment points that puncture the skin lateral to the wound also interfere with long-term cosmesis and provide a possible conduit for infecting microorganisms.

U.S. Pat. No. 5,176,692 to Wilk et al., discloses a device for hernia repair that utilizes mesh with pin-like projections to cover hernia defects. This device, however, is used in a laparoscopic hernia repair in conjunction with an inflatable balloon. Closure devices for deeper tissues are described in U.S. Pat. Nos. 4,610,250 to Green; 5,584,859 to Brozt et al.; and 4,259,959 to Walker. However, these devices either work in conjunction with sutures, are made of materials that do not suggest biodegradability, or are designed in such a way as not to impart uniform tension on the closure, increasing the risk of wound separation and failure of wound healing.

The present invention is a biodegradable tissue approximation device. The device includes a plurality of attachment points, e.g. tines, prongs, or other generally sharp or blunt parts, connected to a backing that can be manipulated to close wounds, join soft tissue or bone, or create anastomoses. This multi-point tension distribution system (MTDS) device may be placed with minimal tissue trauma. The present invention typically incorporates the deeper layers of tissue within the closure, and the multiple attachment points distribute the resulting tension, often uniformly. Approximation from the internal aspect of the wound minimizes the potential for dead space in the closure, thus decreasing the risk of sub-optimal healing. Moreover, because the device is absorbed, a second procedure is not typically needed to remove the device.

Thus, the present invention improves the mechanical phase of healing by facilitating wound closure and/or the coaptation of tissues prior to initiation of the biochemical phase of wound healing. Placement of the device maximizes the chance for a good cosmetic result and is not heavily dependent on surgeon skill. Closure time is also shortened, decreasing overall cost and risk of operative complications.

A variation of the present invention is well suited for inferior orbital rim, craniofacial, and maxillofacial reconstructive procedures.

Current orbital rim, craniofacial, and maxillofacial reconstructive procedures have a number of problems to overcome. The problems to be overcome arise from elevating the soft tissue or skin off the bone repair site. Elevating the soft tissue is generally necessary to access and repair the bone site. Typically, the fractured bones are set using a fracture fixation device such as a biocompatible or biodegradable plate which is attached to the underlying fractured bones using screws.

After the bone site is repaired, however, the soft tissue which was elevated must be re-anchored. Failure to re-anchor the soft tissue results in undesirable sagging or drooping.

Conventional techniques to reduce the sagging and drooping of soft tissue in these regions utilize sutures. Sutures are typically attached to screws or anchors or the bone itself via a drill hole. The soft tissue is then attached to the suture. This conventional technique is undesirable for the reasons set forth above in connection with the use of sutures.

The present invention overcomes the above noted problems by providing the inventive features herein described. In particular, the present invention provides one or more attachment points to hang soft tissue in the orbital, craniofacial, and maxillofacial regions to prevent sagging without the use of sutures. Furthermore, use of the present invention provides a one-step procedure for orbital fracture fixation and tissue approximation or fixation.

FR-A-2 744 623 discloses an elastic device for stretching living tissue. The device includes a flexible elastic part and at least two hooking parts, each of which hooking parts is integral with a base secured to one of the ends of the elastic part. Each base of the hooking parts covers a portion of the elastic part. By moving the bases apart so as to stretch the elastic part, and then hooking the hooking parts into the underside of scalp tissue on opposite sides of an opening in the scalp tissue, the tension in the elastic part will act to draw the bases and their hooking parts, and thus the scalp tissue around the opening, together. The undersides of the bases are moveable with respect to the cranium, so as to enable the elastic restoring force of the elastic part to be used to draw the scalp tissue together.

US-A-5 425 747 discloses a suture made of bioabsorbable material. The suture has a central body member with a plurality of lateral members extending perpendicularly therefrom and in the same general plane with the body member. Each lateral member has a plurality of barb members extending at an acute angle therefrom, allowing the lateral members to be inserted laterally into two sides of a cut in body tissue so that the two sides are joined at an incision junction at the central body member and are retained securely and non-withdrawably in the body tissue by the barb members.

### SUMMARY OF THE INVENTION

According to the present invention there is provided the device of claim 1.

A favoured application for the device of the present invention is the approximation of soft tissue in brow lift and other craniofacial and maxillofacial surgical procedures. Such a device may be optimized to distribute loads over the device while the device remains attached to the patient's cranium. The brow lift device may further include multiple variations of the device and is preferably biodegradable and absorbable by the patient The device may also be made from biological materials. A device variation may be installed into a patient by first creating an incision in the patient's scalp. This incision is preferably a predetermined length corresponding to the length of scalp or tissue desired to be lifted. At one end of the incision, preferably the end farthest away from the scalp or tissue to be lifted, the doctor or surgeon would drill a hole into the cranium. At the opposing end of the incision, the device may be inserted under the scalp or tissue which is then set on the device via attachment points affixed to the device surface. The surgeon may then lift the scalp or tissue via the device, which may then be secured to the cranium by inserting an anchoring post into the drilled hole. Alternatively, after the incision is made and the hole drilled in the cranium, the device may first be inserted into the hole via the post The surgeon may then lift the scalp or tissue into position over the device and then set the lifted tissue onto the attachment points.

In either case, the procedures may be accomplished by a variety of methods. One particularly useful tool may comprise a manipulatable handle having opposing grasping arms. The grasping arms may be used to secure and handle the device via the anchoring post. The tool may include a slidable block which may be angularly disposed relative to the handle so that the block may press down and secure a portion of the scalp or tissue to be lifted. The block is preferably disposed angularly such that the angle of the block is similar to the angle of the attachment points affixed to the brow lift device. Angling the block may allow the tissue to be optimally sent against the attachment points and may provide the least resistance to piercing the scalp or tissue. Alternatively, the tool may omit the slidable block completely and the tissue may be set against the attachment points by other methods such as simply pressing against the tissue by hand.

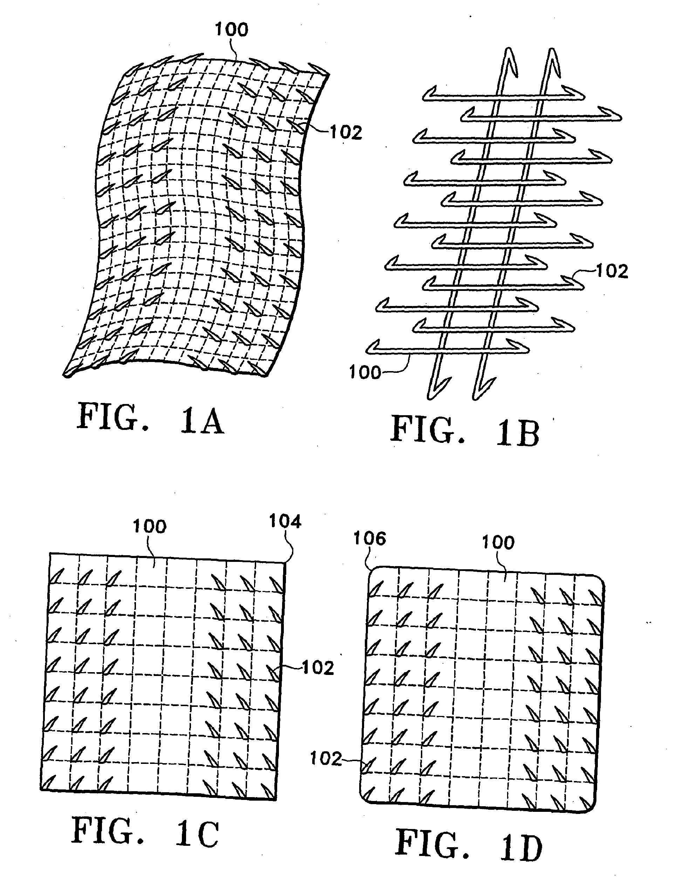

Embodiments of device in accordance with the present invention will now be described, by way of example only, with reference to the accompanying drawings, described below. Although, in the precise form illustrated, the devices of Figs. 1-12, 33, 34, 35A, 35B, 37C, 37D, 38A, 39-40 are not in accordance with the invention as claimed, they will assist the skilled person in understanding the device of the present invention and its use.

### BRIEF DESCRIPTION OF THE DRAWINGS

Figures 1A-1D are plan, perspective views of various MTDS devices.
Figures 2A-2E are side views of various attachment point shapes and orientations.
Figures 3A-3D and 3F-3G are side views of various attachment points.
Figure 3E is a side view of a two-sided MTDS device.
Figure 3H is a plan, reverse perspective view of nubs on the inferior surface of a MTDS device.
Figure 4A is a side, cross-sectional view of attachment points that run through the width of a backing.
Figure 4B is a side view of attachment points on a strip of backing material.
Figure 4C is a plan, perspective view of the embodiment in 4B on a backing.
Figure 4D is a plan, perspective view of attachment points on a solid backing.
Figure 5A is a plan, perspective view of attachment points canted in one direction.
Figures 5B-5D are plan, perspective views of attachment points with various orientations on a backing.
Figure 5E is a side view of attachment points becoming progressively shorter the closer they are to the center of the device.
Figure 5F is a side view of attachment points becoming progressively shorter the farther they are from the center of the device.
Figures 6A-6B are schematic views of a skin wound and wound repair using the MTDS device.
Figure 7 is a schematic view of an abdominal wound closure using MTDS devices.
Figures 8A-8B are schematic views of an abdominal hernia and hernia repair using the MTDS device.
Figures 8C-8D are side and schematic views, respectively, of a MTDS device with attachment points on the edges of the backing and a central area without attachment points.
Figures 9A-9B are schematic views of a ruptured tendon and tendon to bone repair using the MTDS device.
Figure 10A is an axial view of a cross-section of a vessel repaired with the MTDS device.
Figures 10B-10C are side, schematic views of vessel free ends and a vascular anastomosis using the MTDS device.
Figures 11A and 11B-11C are schematic, side, and cross-sectional side views, respectively, of a transected tendon and a tendon to tendon repair using the MTDS device.
Figure 11D is an axial, cross-sectional view of the MTDS tendon to tendon repair.
Figure 11E is a side view of a vascular anastomosis using the MTDS device on the external surface of a vessel.
Figure 11F-11G are side, schematic views, and Figure 11H is an axial view of the ends of a tubular structure being joined by externally placing strips of a MTDS device on approximated tissue.
Figure 11I is an axial view of a hinge in the backing of a device.
Figures 11J-11K are axial views of decreased backing material that are areas of enhanced device flexibility.
Figures 11L-11M are side views of a spring or coil-like MTDS device being used to approximate tissue.
Figure 12A is a schematic view of the MTDS device being used in a brow-lift procedure.
Figure 12B is a plan, perspective view of the MTDS device used in a brow-lift
Figure 13A is a front view of a first embodiment of a MTDS device in accordance with the invention having an integral post or anchor used in a brown-lift.
Figures 13B-13C are a top view and a side view, respectively, of the device of Figure 13A showing the attachment points and integral post.
Figure 13D is a perspective view of the device of Figure 13A.
Figure 13E is a view of cross-section 13E-13E from Figure 13B showing the cavities in the post.
Figures 14A-14D show a top view of a patient's cranium during insertion of the device of Figure 13A.
Figure 15 is a cross-sectional side view of the insertion and securing procedure of the MTDS device from Figure 14C.
Figures 16A-16D are various views of an exemplary attachment point from Figure 13A.
Figure 17A is a view from perspective B-B from Figure 13C of the post having a partial collar.
Figure 17B is a variation of Figure 17A of the post having a full collar.
Figure 17C is a variation of Figure 17A of the post having several tabs.
Figures 18A-18C show back, front, and side views of a post variation missing a distal cavity.
Figure 19A is a perspective view of the post from Figure 18B showing the proximal cavity within the post.
Figure 19B is a view of cross-section A-A from Figure 18B showing the proximal cavity.
Figure 20 is a perspective view of a post variation having a beveled latching mechanism.
Figure 21 is a perspective view of another post variation having an integral beveled latching mechanism.
Figure 22A is a side view of a post variation having a rounded hook.
Figure 22B is a side view of a post variation having an angled post.
Figures 22D-22E are side views of a radially expandable post variation.
Figure 23A is a cross-sectional view of a typical hole in a patient's cranium for receiving a post
Figure 23B is a cross-sectional view of an angled hole variation for receiving a post
Figure 23C is a cross-sectional view of a possible keyed hole variation for receiving a post.
Figures 24A-24C are top, side, and perspective views of a second embodiment of MTDS device in accordance with the invention.
Figure 24D is a view of cross-section 24D-24D from Figure 24A.
Figures 25A-25C are top, side, and back views of a third embodiment of MTDS device which may receive separatable attachment points.
Figures 26A-26C are top, side, and back views of a fourth embodiment of MTDS device having dual tabs on the post.
Figures 27A-27C are top, side, and back views of a fifth embodiment of MTDS device having a latching mechanism on the post.
Figures 28A-28C are top, side, and perspective views of a sixth embodiment of MTDS device having another latching mechanism on the post.
Figure 28D is a view of cross-section 28D-28D from Figure 28A.
Figures 29A-29C are edge, back, and side views of a seventh embodiment of MTDS device having two adjacent posts.
Figures 30A-30C are edge, back, and side views of an eighth embodiment of MTDS device having two aligned posts.
Figure 31A is a top view of a variation of the insertion tool showing the channel.
Figure 31B is a view of cross-section 31B-31B from Figure 31A showing an MTDS device and a side view of the support block.
Figure 31C is a close-up view of the MTDS device and support block from Figure 31B.
Figure 31D is a perspective view from the bottom showing the insertion tool of Figure 31A.
Figure 31E is a perspective view from the top showing the insertion tool of Figure 31A.
Figure 32A is a top view of the insertion tool from Figure 31A showing the block assembly.
Figure 32B is a view of cross-section 32B-32B from Figure 32A showing the MTDS device and a side view of the block assembly.
Figure 32C is a close-up view of the MTDS device and block assembly from Figure 32B.
Figure 32D is a perspective view from the bottom showing the insertion tool of Figure 32A.
Figure 32E is a perspective view from the top showing the insertion tool of Figure 32A.
Figures 33A-33D are front views of devices not in accordance with the present invention.
Figure 33E is a device also not in accordance with the present invention shown in an application.
Figure 34A is another variation of a device not in accordance with the present invention.
Figure 34B is a side view of the device shown in Figure 34A.
Figures 35A is a front view of another variation of a device not in accordance with the present invention.
Figure 35B is a side view of the device shown in Figure 35A.
Figure 35C is a side virus of a modification of the Figure 35A+ 35B device, which modification is in accordance with the present invention and provides a ninth embodiment of the invention.
Figures 36A-36C are front, top, and side views of a tenth embodiment of device in accordance with the present invention.
Figures 37A and 37B are illustrations of an eleventh embodiment of the present invention. Figure 37C,37D + 38A illustrate devices not in accordance with the invention.
Figures 38B is a perspective view of an orbital fastener featuring a plurality of tines on its head, representing a twelth embodiment of the present invention.
Figure 39 is an illustration of an orbital screw and plate not in accordance with the present invention.
Figure 40A is an illustration of a spacer featuring a plurality of tines not in accordance with the present invention.
Figure 40B is an illustration of a spacer, a screw, and a plate combination not in accordance with the present invention.

### DETAILED DESCRIPTION OF THE invention

Our inventive device may be used when working with bone anchors or a variety of soft tissues. Although, as drown it is not in accordance with the invention, the device is of the general configurations shown in Figures 1A-1B and comprises a plurality of attachment points (102) emanating from and preferably affixed to a supportive backing (100) that is a generally a porous material that may have the structure of a mesh, net, or lattice. The degree of flexibility of the backing is determined by the material of construction, the shape and dimensions of the device, the type and properties of the approximated tissue, and the area of the body into which the device is placed. For example, a tightly curved or mobile part of the body, e.g., a joint, will require a more flexible backing, as would a tendon or nerve repair due to the amount of bending the device needs for the attachment. Also, depending on the type of material used, the thickness of the backing as well as its width and length may determine the flexibility of the device. Furthermore, the backing may be pre-fabricated into different shapes as shown by the sharp corners (104) and rounded corners (106) in Figures 1C and 1D. The fabricated cross-sectional shape and dimensions of the mesh elements may vary to promote flexibility in regions of the backing. The cross-sectional shape of the mesh elements may be chosen to minimize local compressive stress between the backing and surface it rests upon, or have rounded and filleted edges to be less obtrusive to local circulation. The plurality of attachment points distribute tension over the contact area between the device and the tissue. The tension or forces are generally also distributed in the tissue and in the backing parallel to the interfaces between the tissue and the device.

Materials such as biodegradable polymers are preferably used to construct the backing and attachment points. Polymers synthesized from monomers comprising esters, anhydrides, orthoesters, and amides are particularly suitable for biodegradation. Examples of biodegradable polymers are polyglycolide, polylactide, poly-α-caprolactone, polyglyconate, polylactide-co-glycolide, and block and random copolymers of these polymers. Copolymers of glycolic, lactic, and other α-hydroxy acids are highly desirable. Although we prefer to use a single polymer or copolymer in a specific device, generally for ease of construction, the invention is not so limited. An example of an inventive device may be made of two or more types of polymers or copolymers (or molecular weights of the same polymer or copolymer). For instance, the backing material might be produced from a more flexible polymer and the points or tines of a stiffer material. The inflammatory response to these polymers is minimal, and they have been safely used in suture materials, stents, drug delivery devices, orthopedic fixation devices, and intestinal anastomotic rings.

Generally, we will refer to the attachment points as "tines" or "prongs". These tines, will refer both to points which are either sharp, i.e. able to separate tissue in a chosen use, or blunt, i.e. not able to separate tissue in that use. The attachment points may also be referred to as "barbs"'when those points have the retaining point shown in several of the Figures discussed below. Generally the tines, prongs or barbs penetrate into soft tissue and for a short distance. The attachment points preferably do not traumatize tissue in any major way, e.g., by penetration through a selected area of tissue to meet another device on the opposite side of the tissue. For instance, the attachment points generally do not penetrate the subject soft tissue more than 2.54 mm (0.100"). The attachment points may be considered to interlock with modulation in the adjacent soft tissue rather than penetrate as by a pin or bolt.

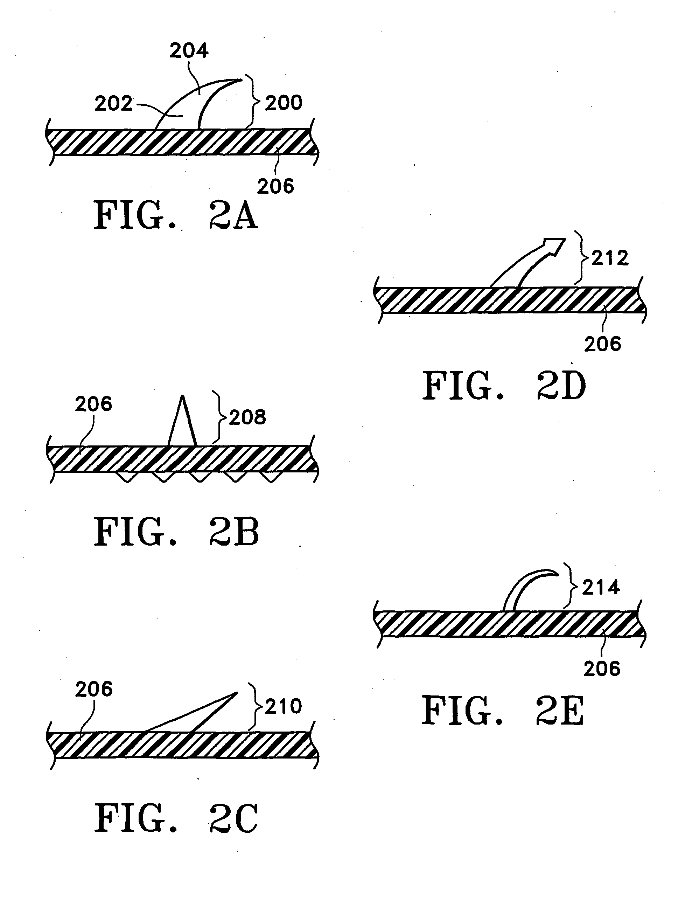

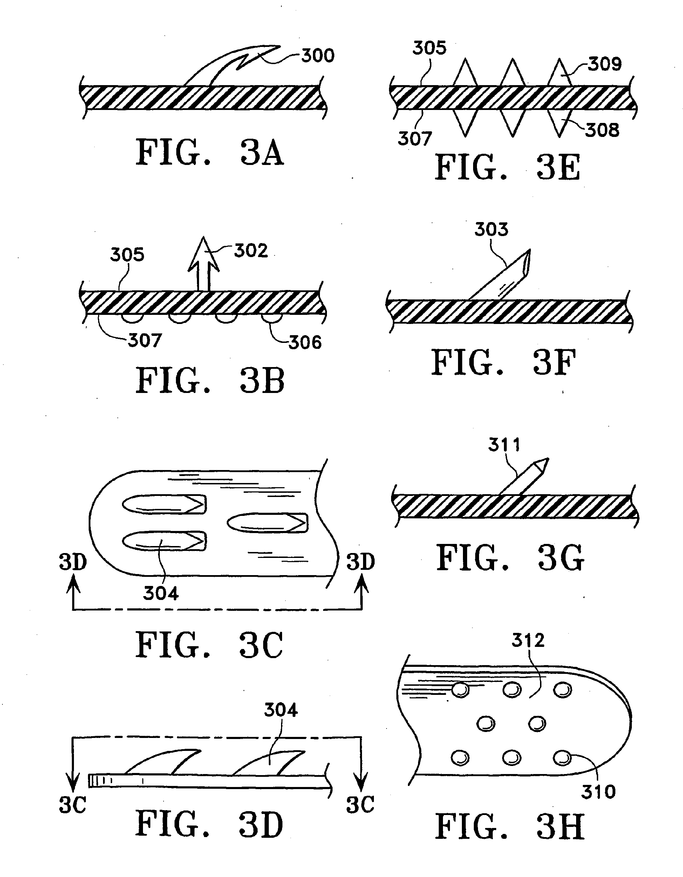

As shown in Figures 2A-2E, the shape of the Attachment points or barbs may be varied depending, e.g., on the area of the body involved and the type of tissue requiring closure or reapproximation. The tines may be canted or erect and maybe with a thorn shape. As shown in Figure 2A, the tines (200) have a wide base (202) that supports a projection (204) from the backing (206) against the degree of tension required to close a wound or approximate tissue. For example, the attachment points may be erect tines (Fig. 2B-208), canted tines (Fig. 2C-210), canted arrowheads (Fig. 2D-212), canted hooks (Fig. 2E-214), or may have a single straight cross-section (Fig. 3G-311) that is nail-like, that does not vary over the length of the prong, for example, similar in shape to a nail or sharpened pencil. Furthermore, the tip of the attachment points may be varied as shown in Figures 3A-3D. The tips may be barbed (300 in Figure 3A), arrowhead (double-barb) (302 in Figure 3B), or cheese grater (304 in Figure 3D). A side view of the cheese grater tips is shown in Figure 3D. A faceted tip (303 in Figure 3F) is shown. The faceted tip is especially desirable where the force to penetrate tissue is normal to the tissue surface.

The connection of the prong to the backing may be rounded or filleted, or the backing built-up around the prong, to reduce structural stress concentrations. The backing or connecting structure may branch out away from the center, with each branch in turn branching to grapple tissue in a distributed fashion. All edges of the device may be smooth except where sharpness is needed at the tip of the prong to pierce into the tissue. Once the prongs pierce into the tissue, the tissue may become supported against the backing to minimize additional piercing or irritation by the prong tip. The device may be molded, stamped, machined, woven, bent, welded or otherwise fabricated to create the desired features and functional properties.

The MTDS device may also have attachment points both on its front side (305) and on a back side (307). As shown in Figures 3B and 3E, the front and back sides have attachment points. The attachment points on the front side (309) generally approximate tissue. The attachment points on the back side (307) are auxiliary attachment points that may comprise forms such as round nubs (306) or pointed nubs (308). The auxiliary attachment points may be used to secure or promote stable implantation of the device. Soft tissue may be gently pressed into open regions of the backing thereby helping to fix the device in place against both underlying and overlying tissue after the modulation or interlocking of skin. Figure 3H shows a reverse view of the nubs (310) on the back side of the device (312). The attachment points on a two-sided device are not limited to the combinations disclosed above, but may comprise any combination of the previously mentioned attachment point shapes and orientations.

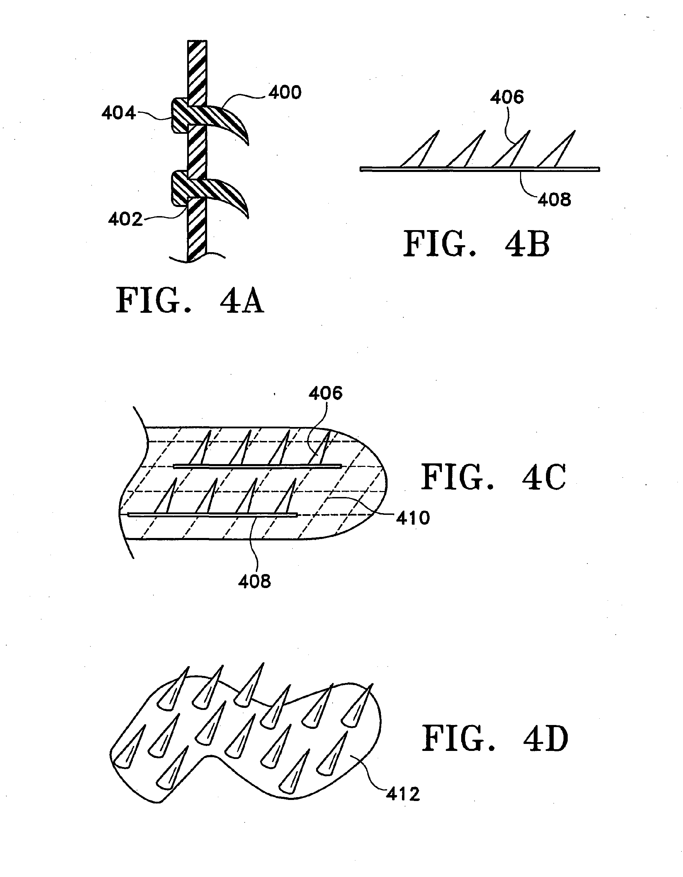

Structural variations can also be made to the backing of the device. As shown in Figure 4A, the attachment points (400) may be placed through a plurality of openings in the backing (402) and secured to the backing by a flange (404) or hub. In Figures 4B and 4C, the points (406) may also connect to strips (408) of the same material as the attachment points which are then secured to a backing (410). The backing may also be comprised of a solid material (412) instead of a porous material.

The extent of porosity, or total surface area may be used to control the absorption rate of the device, and may also be used to optimize the strength-to-mass properties of the device, increasing the section modulus of structural cross-sections per unit mass. The backing structure may comprise partial folds, waves or grooves to help hold tissue against both surfaces of the backing. Regions of the backing may function as suction cups to help hold tissue to the backing.

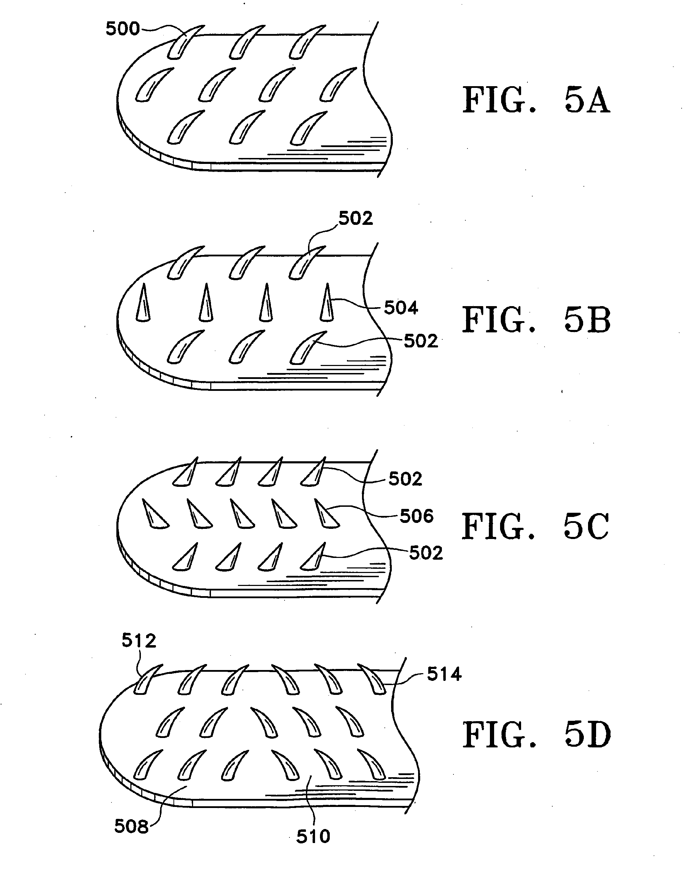

The density, distribution, length, and orientation of attachment points on the backing may be modified depending on the type of wound closure. Attachment points may be bent or curve gradually, with the tip directed at an optimal angle relative to the backing to aid device penetration and stability within the tissue, and to reduce tissue irritation after device installation. Attachment points may be canted in one direction (500), such as toward the center of the device as shown in Figure 5A. The attachment points may also be variously oriented, such as toward center (502) and erect (504), or toward center (502) and away from center (506). It is within the scope of this invention to have attachment points extending in any relative direction or orientation on the backing. Or, as shown in Figure 5D, the backing is divided into a first area (508) and a second area (510). Attachment points in the first area (512) and second area (514) are canted toward each other. The inventive device may also be sectioned into a plurality of areas, with each section being variously oriented to another section.

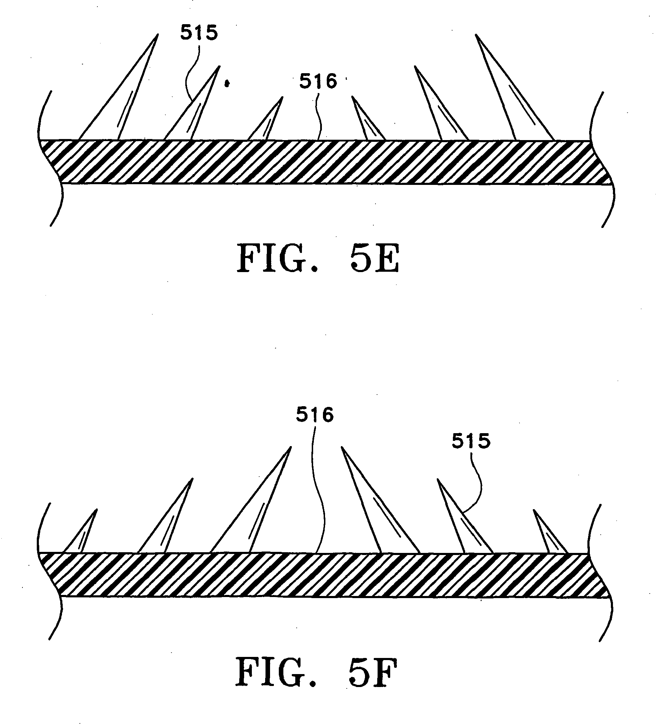

Attachment points of various lengths may emanate from a single backing. For example, in Figure 5E, the attachment points (515) are progressively shorter the closer they are to the center of the device (516). The attachment points (515) may also become progressively shorter the farther they are from the center of the device as shown in Figure 5F. The variations shown in Figures 5B and 5C have regions of attachment points canted toward the center (502) and with other regions of attachment points with erect points (504 in Fig. 5B) or canted away from the other end (506 in Fig. 5C) of the device. These variations are more difficult to dislodge when situated in an area of the body having both to-and-fro movement, e.g., the inside of an elbow or back of the knee, or during placement of the device.

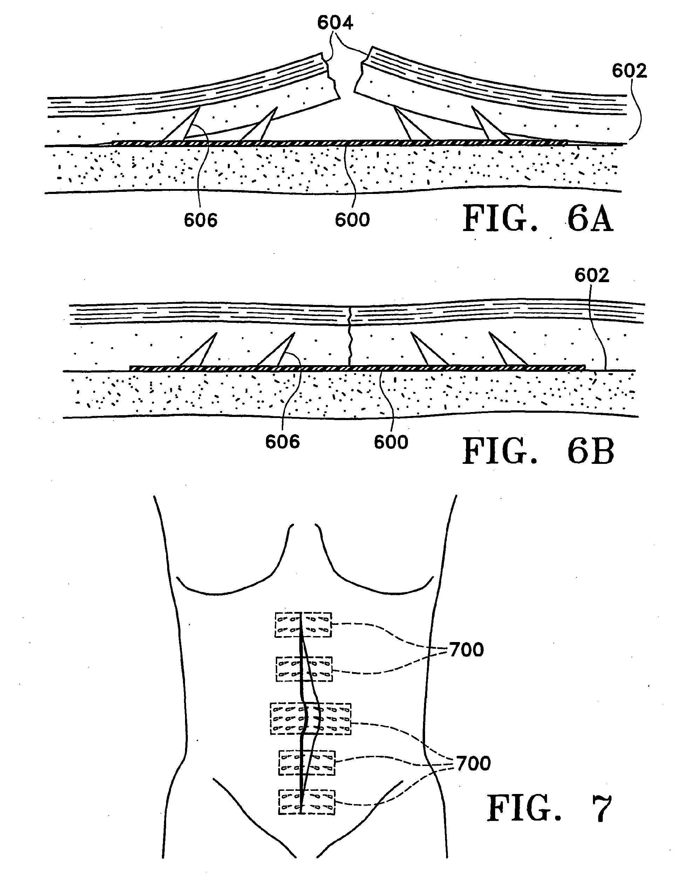

Portions of simple wound closures are shown in Figures 6A-6B. These wound closures involve placing the MTDS device (600) at the bottom of the wound, usually at the level of the sub-dermis (602). The edges of the wound (604) are approximated and then secured by fixation, e.g., by pressing, to the multiple attachment points (606). An example of the MTDS device placement in a laparotomy closure is shown in Figure 7. The increased length of this incision requires placement of multiple devices (700).

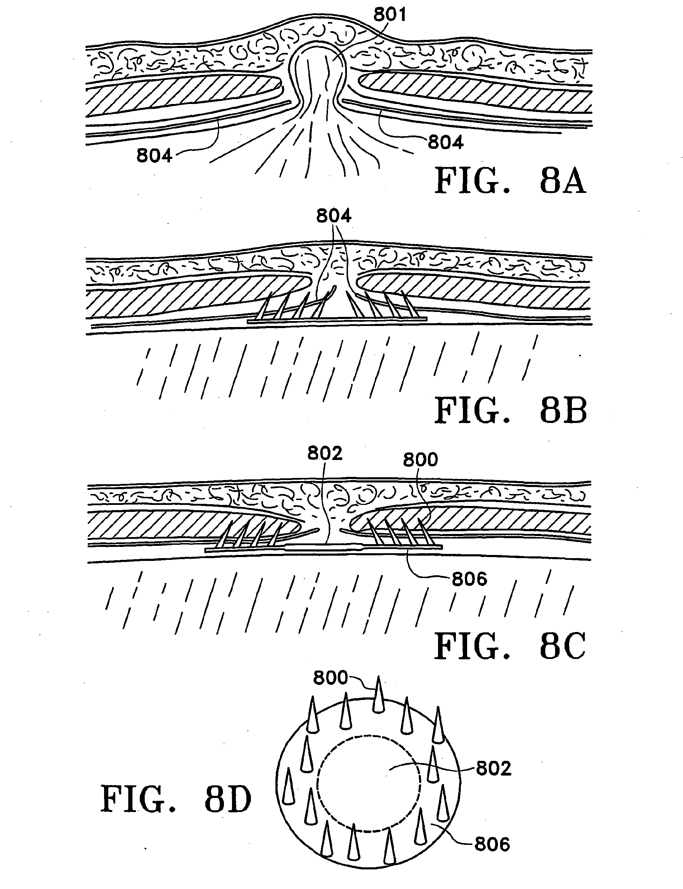

A unique application of this device occurs in hernia repair in which case the biomaterials are not absorbable but rather are more likely to be PTFE and POPU ("Gore-Tex"), polypropylene, or other permanent implant material. Once the hernia (801) is reduced, a MTDS device may be used to close the hernia defect by joining the edges of the separated fascia (804) as seen in Figures 8A and 8B. However, the device may also be modified to aid repair of a difficult hernia resulting from such circumstances as operating on an obese patient or large hernia, or having a wide fascial debridement where the fascial edges cannot be brought together. Figures 8C and 8D are variations of the inventive device that may be used in these cases. The attachment points (800) are secured to the ends of the backing (806) and are still used to adhere the device to tissue, but the points are spaced so that the central area of the backing is a flat surface without points (802) that covers the defect. The device in Figure 8D is preferably used in an incisional hernia repair.

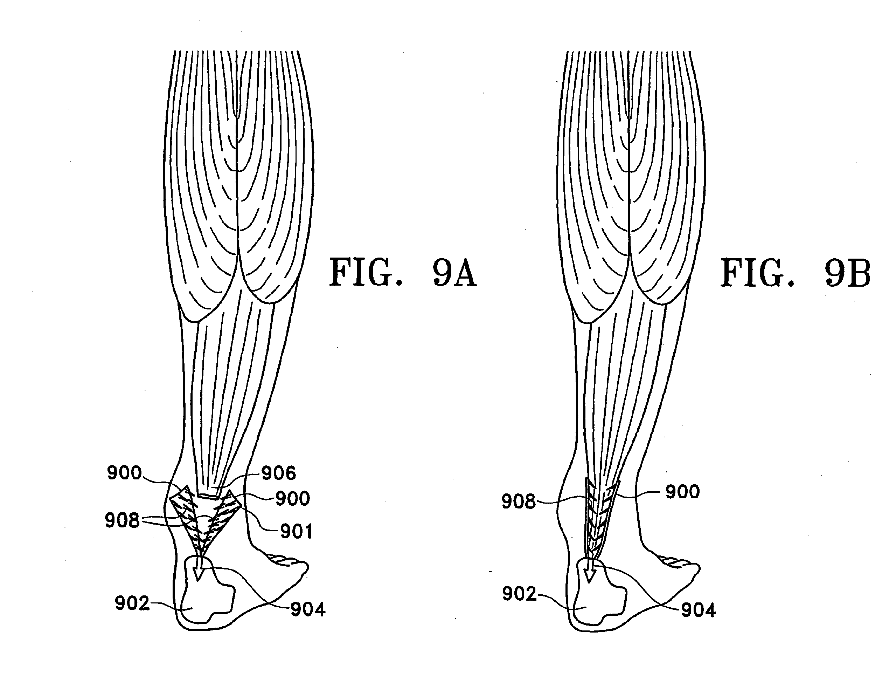

The MTDS device may also be constructed to reattach soft tissue such as tendons and ligaments to bone, as well as other soft tissue such as cartilage and the free ends of vessels or nerves. In Figure 9A, the inventive device functions similar to a clamp. Backings with attachment points (900) are sides of a clamp that has a first end (901) and a second end (904). The first end (901) grasps tissue and the second end (904) is an anchor for tissue. For example, a ruptured tendon (906) may be fixed to the attachment points (908) of the first end of the clamp (901) and approximated to bone (902) with an anchor such as a pin or nail at the second end of the clamp (904), as seen in Figure 9B. After mechanical fixation of the tissues, the biochemical phase of the wound healing process will begin, eventually forming a natural union between tendon and bone. Ligament and cartilage to bone unions using the MTDS device would undergo the same mechanical and biochemical processes.

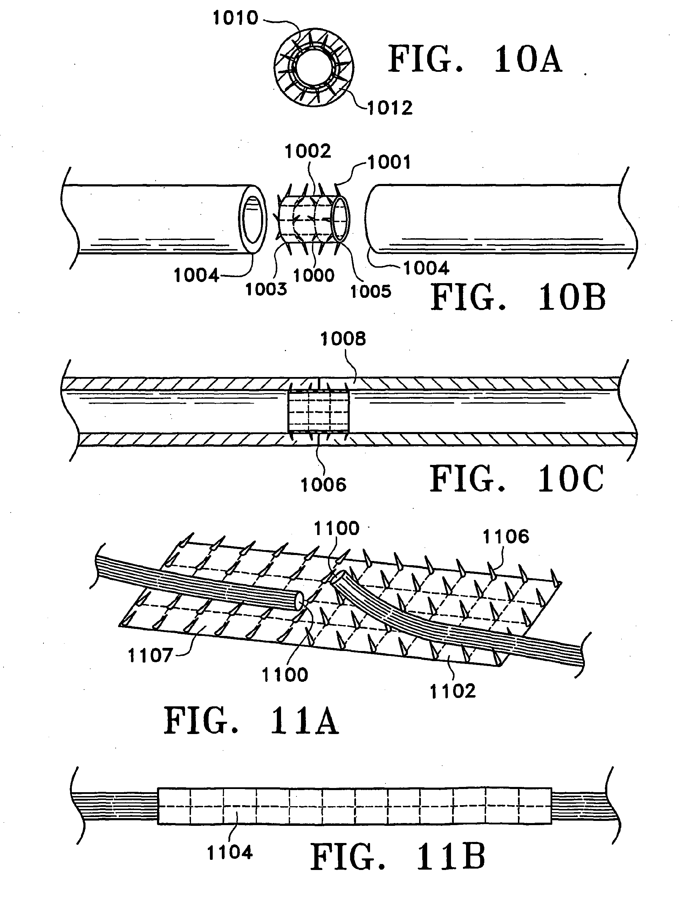

Vascular anastomoses may also be constructed with the MTDS device. In Figure 10B, the basking has a tubular shape (1000) with attachment points (1001) on the outside surface (1002). The outside surface (1002) has a first end (1003) and a second end (1005) that opposes the first end (1003). The free ends of a vessel(s) (1004) are placed over the device, creating an anastomosis (1006) that is secured by attachment points fixed into the wall of the vessels (1008). The attachment points are preferably pointing towards the anastomosis (1006), with the attachment points on the first end (1003) being canted toward the second end (1005) and vice-versa. An axial view of the relationship of the attachment points (1010) to the vessel wall (1012) is shown in Figure 10A.

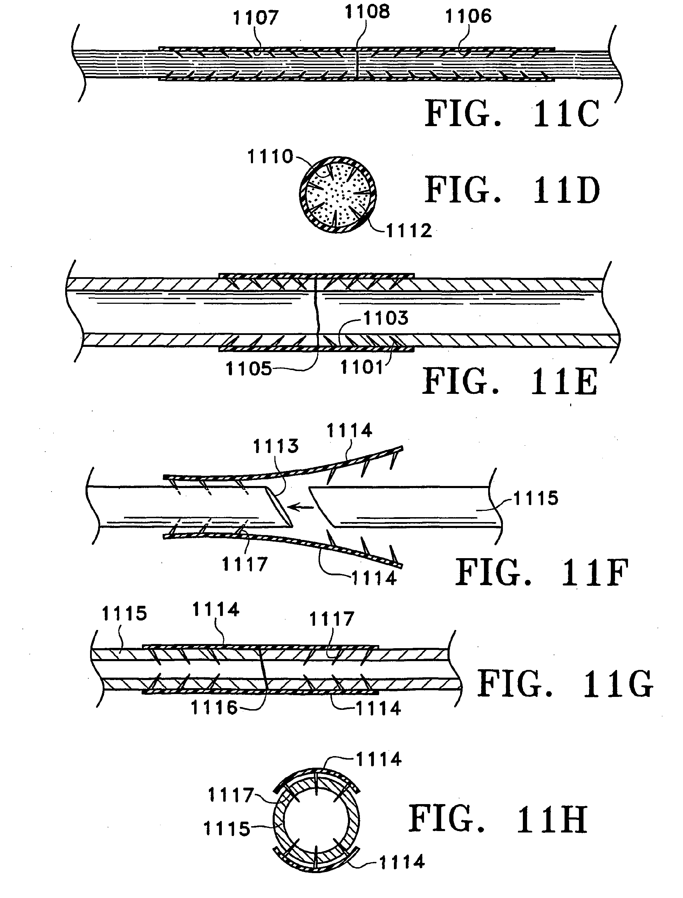

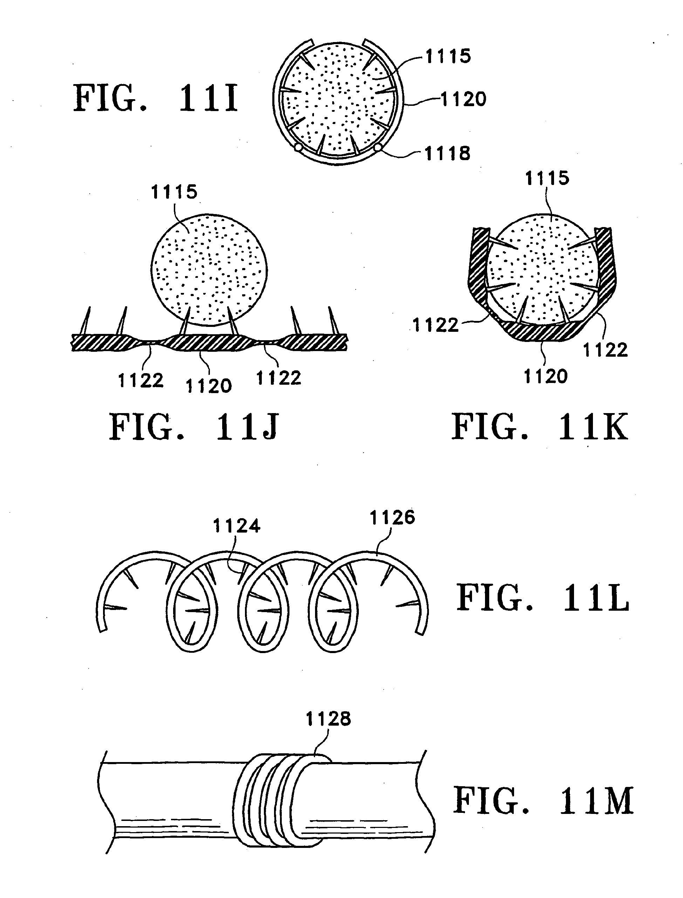

Vessels and other soft tissue such as nerves, cartilage, tendons, and ligaments may also be joined as seen in Figures 11A and 11B. Two ends of tissue (1100) are brought and held together by the backing and attachment point construct (1102) being wrapped around the circumference of the tissue (1104). The attachment points (1106) are on the inside surface of the backing (1107) and secure the union at a central region (1108) as seen in Figures 11C. An axial, cross-sectional view of the relationship between the attachment points (1110) and tissue (1112) is shown in Figure 11D. The resulting form is, i.e., a tubular structure that has an inside surface (1107) with a central region (1108). The attachment points on the inside surface (1106) may be canted toward the central region (1108). Figure 11E shows the device with attachment points (1101) on the inside surface of the backing (1103) being wrapped around vessel ends to create an anastomosis (1105). Instead of being wrapped around tissue, edges (1113) of tubular structures (1115) can also be joined by externally placing 2 or more strips of backing of a MTDS device (1114) on approximated tissue as shown in the side views of Figures 11F-11G, and the axial view in Figure 11H. The attachment points (1117) also point toward the area of tissue approximation (1116).

Figures 11I-11M illustrate ways of varying the mechanisms used to improve device flexibility. In Figures 11I-11K, the backing has areas of comparatively higher flexibility than other areas of the backing. In an axial view of the variation in Figure 11I, the backing is equipped with hinges (1118) that allow bending of the backing (1120) around tubular soft tissue structures (1115). In a second variation, the amount of material in the areas of the device that fold (1122) is reduced as shown in Figures 11J-11K. Another variation is seen in Figures 11L-11M where attachment points (1124) of a device extend from a backing in the form of a coil or spring (1126). The edges of soft tissue are approximated when the coil or spring is reduced (1128).

### Device for Brow and Face Lift Procedures

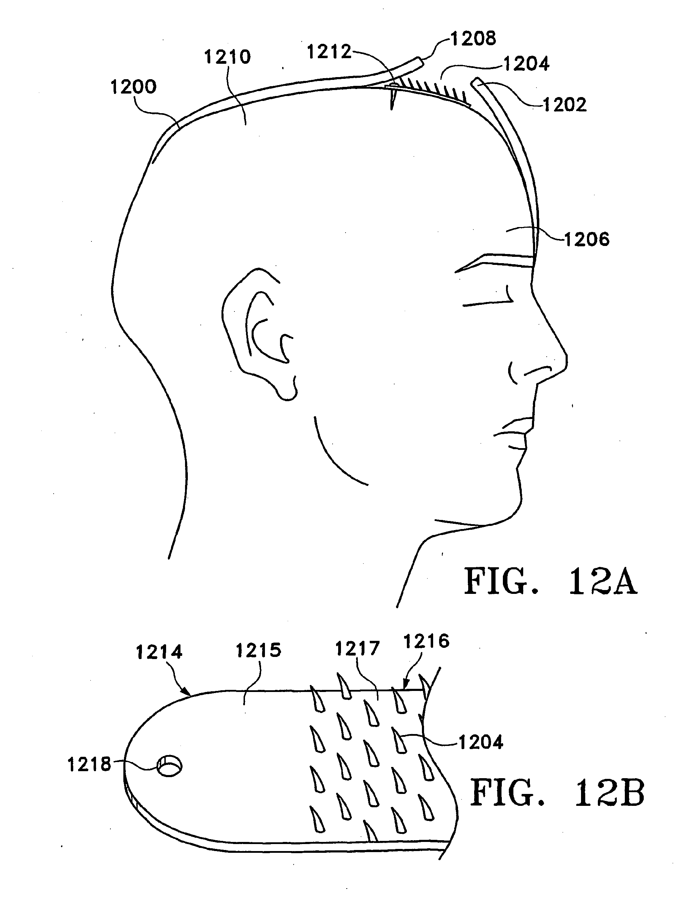

The device may also be used in soft-tissue remodeling, such as a brow-lift, shown in Figure 12A. After dissection of the scalp (1200), the anterior scalp flap (1202) may be raised over the attachment points (1204) to lift the brow (1206). The ends of both the anterior flap (1202) and posterior flap (1208) may then be trimmed and fixed onto the attachment points (1204) to close the wound. The device may be secured to the skull (1210) by a screw (1212). The inventive device in this example may have a first end (1214) and a second end (1216), the first end having a first area (1215) and the second end having a second area (1217). The first area (1215) and second area (1217) may have extending attachment points (1204) or one or more openings (1218) to accommodate a screw(s) (1212). The second area attachment points are canted toward the first end of the device as shown in Figure 12B.

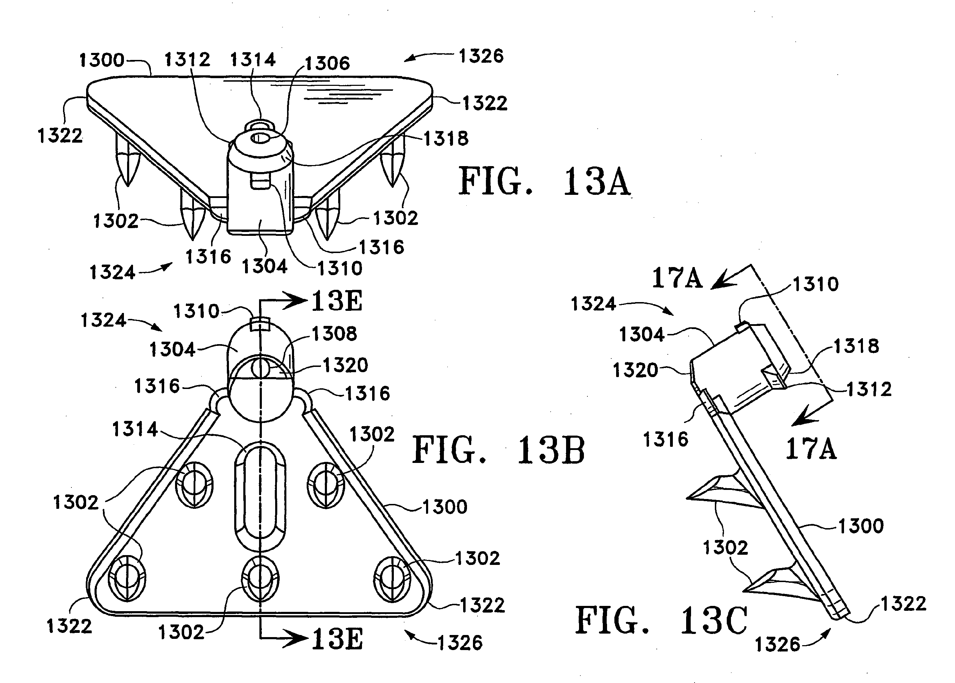

Figures 13A-13C show a first embodiment of device which may be used in a brow-lift or similar surgical procedure. This device may generally be inserted under a patient's scalp while securely interlocking a small portion of the scalp to the device preferably via a plurality of attachment points. It may also be designed generally to lay against the cranium in a low profile while secured to the cranium to provide a brow lift. This device comprises supportive backing (1300), which is shown substantially as an equilateral triangle, or in a delta shape. Backing (1300) may be any of a wide variety of triangular shapes, e.g., isosceles, etc. which functions to distribute planar loads equally radiating from a small area, e.g., post (1304). Various alternative shapes are discussed below in greater detail. Post (1304) is functionally for the maintenance of the device in place; other sections of the surgical procedure used to support the device in a specific part in the body. Post (1304) is placed on the side of the body opposite to the tines.

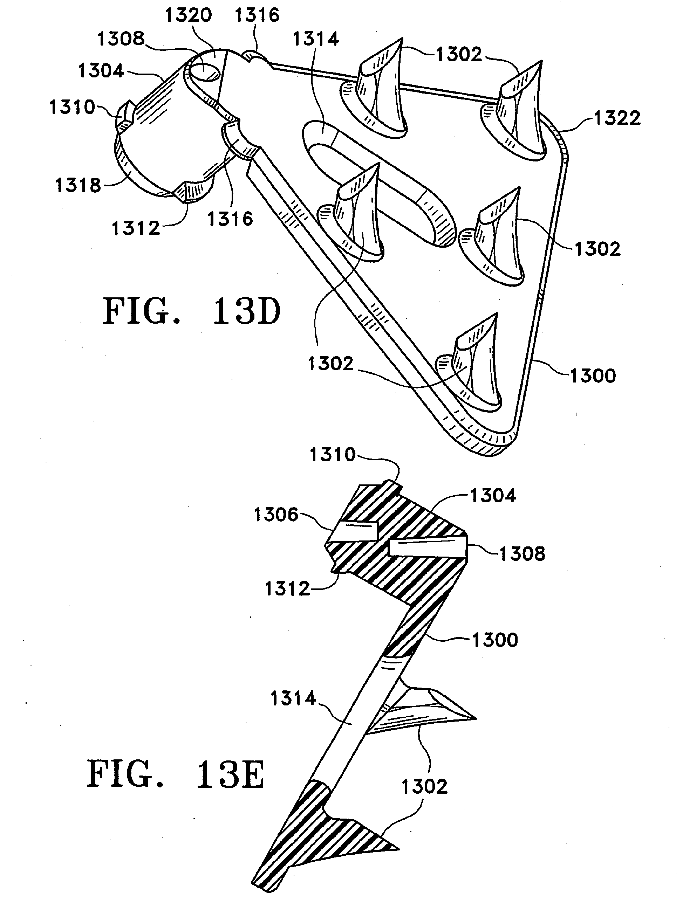

Figure 13A shows a front side view of supportive backing (1300). This may incorporated sharp corners at the triangle vertices, but preferably has radiused or rounded corners (1322) to aid in reducing abrasion and cutting in adjacent tissue. Anchoring post (1304) may be located at one of the vertices of backing (1300). This anchoring post (1304) is shown as being substantially perpendicular to a plane of backing (1300), but may be other shapes as discussed below. Moreover, this device may be made of any of the materials discussed herein, and is preferably comprised of a biodegradable or bioabsorbable material but is obviously not limited by material type. For instance, the device may be comprised of certain biological materials as well, e.g., collagen, hydroxyapatite from both natural and synthetic sources, bone graft, or any combination or polymerized version of these materials. Figure 13D shows more clearly a perspective view of a preferred variation of the device shown in Figures 13A-13C.

In this first embodiment of device supportive backing (1300) may comprise a triangular form having a first end (1324) and a second end (1326). This is typically comprised of a front side, as shown in Figure 13A, and a back side, as shown in Figure 13B. On the front side, preferably near a vertex of the triangular shape, is an anchoring region. This region may comprise anchoring post (1304) as seen in Figures 13A-13C, and this anchoring post (1304) may be a variety of shapes, e.g., a hook or an angled post, etc., but is preferably a perpendicular post having a proximal and a distal end. Moreover, post (1304) is preferably integral with backing (1300) so as to be formed from a single piece. This allows the device to be formed entirely into a single integral device by various manufacturing methods, e.g., injection or die molding. Post (1304) may also be a separate structure fixedly attached to backing (1300) by any variety of fastening methods, e.g., mechanical fasteners or adhesives. The distal end of post (1304) may be chamfered (1318), as shown in Figures 13A and 13C; this would provide a degree of tolerance to enable the surgeon to easily locate and insert post (1304) into a receiving hole without sacrificing device integrity.

Post (1304) may preferably further comprise a locking device proximal of chamfer (1318). This locking device may utilize a variety of locking mechanisms but is shown in this variation as front tab (1310) and partial collar (or rear tab) (1312). The locking mechanism is preferably integral with post (1304) and may have a diameter which is greater than a diameter of post (1304). In any case, partial collar (1312) is preferably elastically deformable, but may also be plastically deformable. Such deformability allows front tab (1310) and partial collar (1312) to compress upon insertion into a patient's skull and subsequently be able to spring back upon full insertion to provide a friction-fitted locking or securing feature. The locking device may alternatively be a locking key mechanism or any conventional locking mechanism. However, the locking mechanism may be omitted entirely because the device bases much of its stability, once inserted into a patient's cranium, upon the downward forces applied by the overlying tissue. Thus, much of the forces acting on the device apply bending loads on post (1304) rather than axially-oriented tensile loads.

As seen in Figure 13A, post (1304) may incorporate a distal channel or cavity (1306) which may extend partially into the post from the distal end or entirely through the post. This distal cavity (1306) may have a diameter which is smaller than the diameter of post (1304) and may be aligned along an axis defined by post (1304) or may extend at an angle within post (1304). The cross-section 13E-13E of Figure 13B is shown in Figure 13E and shows more clearly the orientation of distal cavity (1306) within post (1304) for this variation. Distal cavity (1306) may aid in reducing the amount of material used in the manufacture of the device, and is particularly useful in imparting a desirable degree of flexibility to post (1304) which may facilitate the insertion of post (1304) into the cranium.

Post (1304) may further define another hole, proximal cavity (1308), which may be used for tooling purposes as well as further adding to the flexibility of post (1304). Proximal cavity may extend from chamfered proximal end (1320), which may also aid in tooling and helping to prevent tissue abrasion. Proximal cavity (1308) may be non-concentrically located relevant to distal cavity (1306) and as shown in Figure 13E, may extend partially into post (1304) or may be a through-hole extending entirely through to the distal end of post (1304). Although proximal cavity (1308) may not necessarily be required, it may be utilized in a variety of ways. For example, proximal cavity (1308) may be used for aligning the device for tooling during manufacture, or it may also be used as a location to allow a user or surgeon to manipulate the device using tools for placement of the device within a patient This proximal cavity (1308) may have a diameter, e.g., about 1 mm, which is smaller than a diameter of post (1304).

In addition to proximal cavity (1308), the device may also comprise protrusions, tabs, or "ears" (1316), as seen in Figures 13A-13D. These protrusions (1316) are preferably integral with backing (1300) and may generally be located anywhere on backing (1300), but is preferably located near first end (1324), and more preferably near post (1304). Figure 13B shows protrusions (1316) located on either side of post (1304) and may provide a surface for manipulating the device by the doctor or surgeon either during placement into the patient or during removal.

Figures 13A and 13C show the front and side views, respectively, of attachment points (1302). As discussed above, attachments points (1302), also called "tines" or "prongs" are preferably integrally affixed to backing (1300) but may also be separately attachable. They are preferably located on the back side of backing (1300), i.e., the side opposite of post (1304), and are preferably angled towards first end (1324). Moreover, individual attachment points (1302) may be of varying sizes and angles depending upon the desired securing effect. Attachment points (1302) are discussed in greater detail above. In this variation, individual attachment points (1302) may vary in density, but are optimally spaced relative to one another. Factors for optimizing attachment point relative placement may comprise the ease of securing tissue to attachment points (1302) and the distribution of loads generated by the attached tissue over each of attachment points (1302). For instance, if attachment points (1302) were located too closely to one another, piercing the tissue would be difficult because of the distribution of stresses on the tissue to be pierced by attachment points (1302).

Another example may include having an increasing number of attachment points (1302) placed on backing (1300) the farther they are located from post (1304) or front end (1324), where the greatest number of attachment points are located in the direction of tensile loads on the device. The spacing between individual points (1302) may be functional in that the number, density, and placement of points (1302) are optimized to evenly distribute the loads, e.g., shearing forces and bending moments, generated by the attached scalp in a brow-lift procedure. Moreover, attachment points (1302) are preferably configured to penetrate partially through the soft tissue. For instance, the sharpness of attachment points (1302) are such that they allow easy penetration through the periosteum.

Figures 13B and 13D show supportive backing (1300) which may also comprise through-hole (1314) that is defined within backing (1300). Through-hole (1314) may generally be any shaped hole but is shown in this variation as being slotted. Through-hole (1314) serves several functions which may include reducing the amount of material used in manufacturing the device, it may also add desirably to the flexibility of backing (1300). Additionally, through-hole (1314) may be configured as an alignment aid for tooling purposes. In addition to aligning, through-hole (1314) may also serve as a surface for a tool to grasp during device placement or removal. Flexibility is preferable because it enables backing (1300) to bend and conform more closely to the shape of the patient's cranium against which the device is placed. The degree of flexibility of backing (1300) may be tuned to a predetermined degree depending upon several factors, e.g., the configuration and size of through-hole (1314). Although shown as a slot, backing (1300) may define virtually any through-hole shape which serves the functions discussed above, i.e., increasing backing (1300) flexibility and aiding in tool alignment.

### Method of Installing and Securing

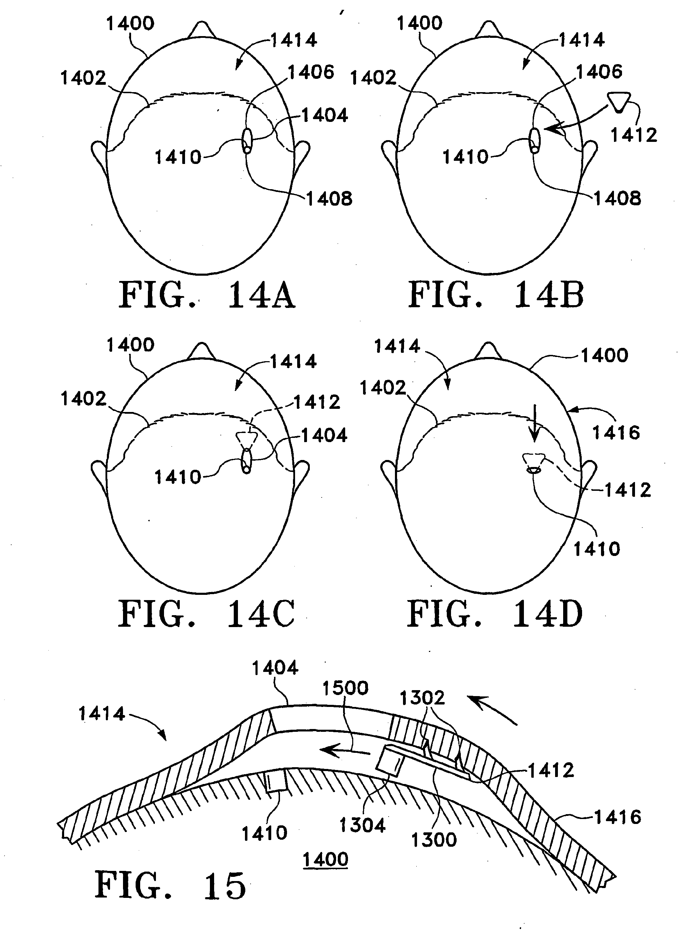

Figures 14A-14D illustrate a preferable method of installing the device of Figure 13A. The top of a patient's head is shown having a hairline (1402). As seen in Figure 14A, the doctor or surgeon may initially make an incision (1404) in scalp (1414) preferably along a sagittal plane defined by cranium (1400). The incision (1404) may typically be done in the patient's hairline, if possible, to minimize any visible scarring which may result. The length of incision (1404) is typically determined by the length or amount of scalp the patient may desire or the surgeon may determine necessary to be lifted for a successful brow-lift procedure. This incision length may generally range from about 1 to 2 cm but may be more or less depending on the desired results.

Once incision (1404) is made, a hole (1410) may be drilled within cranium (1400) at the incision second end (1408). Hole (1410) drilled into cranium (1400) may typically be about 4.0 mm in diameter and may be made by a conventional surgical drill (not shown). As shown in Figure 14B, once the incision and hole are made, an MTDS device (1412) may be inserted between cranium (1400) and scalp (1414) at the incision first end (1406) such that post (1304) faces towards cranium (1400) and attachment points (1302) face the underside of scalp (1414), i.e., subperiosteal. Figure 14C shows an outline of device (1412) placed at incision first end (1406) and beneath scalp (1414). Once device (1412) has been inserted, the portion of the scalp tissue to be raised (1416) is set on device (1412) via attachment points (1302). Figure 15 shows a cross-sectional view of Figure 14C where the tissue to be raised (1416) has been set on attachment points (1302). Once tissue (1416) is set, a force (1500) may be applied to device (1412) preferably via post (1304). Force (1500) then draws the device (1412) and tissue (1416) towards hole (1410) which is configured to receive post (1304). As shown in Figure 14D, once post (1304) is secured within hole (1410), force (1500) may be removed, thereby leaving the brow desirably lifted.

Once device (1412) has been installed, attachment points (1302) and post (1304) undergo shear and bending loads from the lifted tissue (1416) pulling on the device (1412). However, these loads may decrease rapidly and approach zero as scalp (1414) heals. This decrease in loading may take up to about six weeks, but device (1412) may stay in place beneath scalp (1414) for up to several years, with sufficient strength for about six weeks, to prevent scalp (1414) from moving excessively during the healing process and thereafter being absorbed by the body, thereby removing the necessity for a second procedure to remove device (1412).

### Variations on Attachment Points

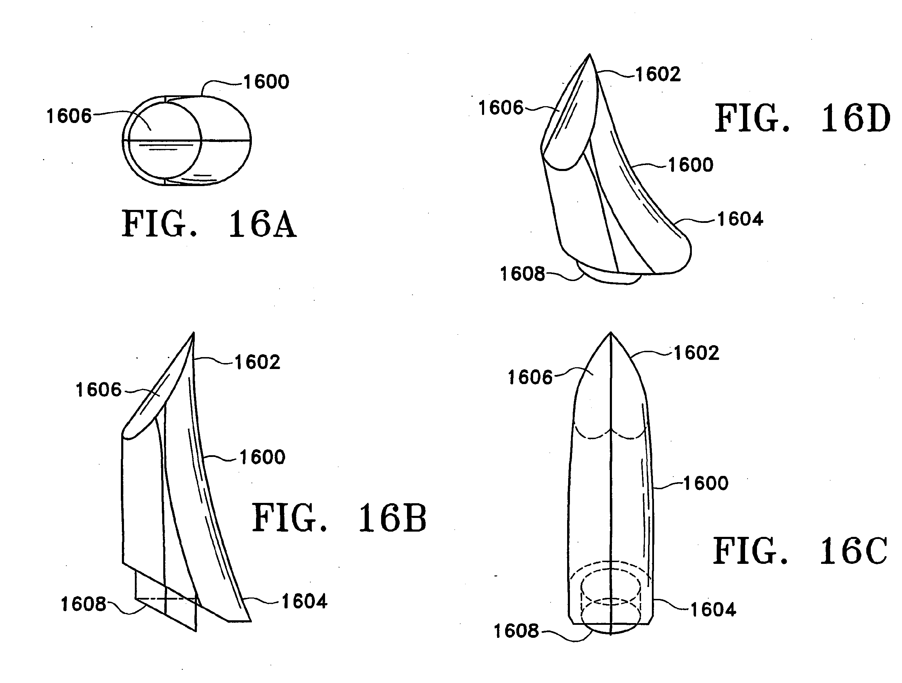

Figures 16A-16D show a preferred variation for an attachment point on a brow lift device. Figure 16A shows a top view of a single attachment point (1600) having a swept face (1606). Figure 16B is a side view of attachment point (1600) comprising distal pointed end (1602) and proximal base end (1604). Although any variations of attachment points discussed above may be used on the brow lift device, this variation is preferable because it is able to readily pierce tissue through the periosteum and simultaneously secure the tissue solidly by resisting any bending moments. In particular, swept face (1606) may be specifically faceted so that face (1606) is preferably oriented to be essentially perpendicular to the plane of the tissue or scalp being penetrated, even though the tine axis defined by attachment point (1600) may not be perpendicular to the plane of the tissue or scalp.

Attachment points of this variation may optionally be manufactured individually and separately from the supportive backing and then individually attached via backing attachment (1608) to the backing by a variety of fastening methods, e.g., friction fitting, adhesives, etc. Optional backing attachment (1608) is seen in Figure 16B, and more clearly in the back view of Figure 16C. Figure 16D shows the variation more clearly in a perspective view. Attachment point (1600), as mentioned, may be manufactured separately and attached, but it is preferably made integral with the MTDS device. Integrating the attachment point(s) (1600) with the backing not only provides uniformity in material type but also eliminates contact interfaces, which in turn may provide superior material strength and resistance to bending.

As discussed above and as shown in Figures 13A-C, attachment points (1600) are preferably manufactured or attached so that they are all substantially canted in parallel towards the post. However, the attachment points are faceted such that the tips of attachment points (1600) are effectively perpendicular to the tissue to be penetrated. Attachment points (1600) may also be manufactured or assembled so that they point in different predetermined directions, depending on the desired application. Furthermore, attachment points (1600) may optionally be made of varying sizes, as discussed in further detail above.

### Variations on Posts

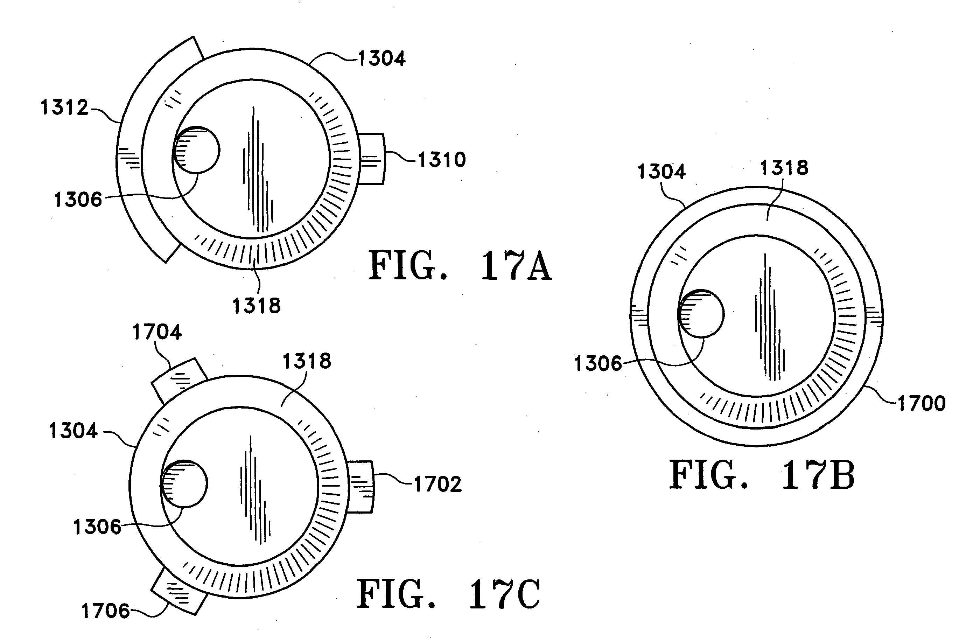

Figure 17A shows perspective 17A-17A from Figure 13C of the distal end of post (1304). As shown, partial collar (1312) and front tab (1310) preferably comprises integral extensions or protrusions which act as a locking device. Both partial collar (1312) and front tab (1310) may be plastically deformable but is preferably elastically deformable. The protrusions provide opposing forces upon insertion into the skull to produce a friction fit which secures the device in the patient Partial collar (1312) may essentially circumscribe any predetermined percentage of the circumference of post (1304), provided that a sufficient fit is produced.

Aside from partial collar (1312), post (1304) may alternatively use locking mechanisms comprising barbs and sub-cortical wings. Moreover, post (1304) may also be threaded so as to be rotated, or screwed, into a threaded mating hole located within the patient's cranium.

Figure 17B shows an alternative locking configuration from Figure 17A. Here, partial collar (1312) is replaced by full collar (1700), which is preferably integral with post (1304) and may also be plastically or elastically deformable. A further variation for a locking configuration is shown in Figure 17C, in which first, second, and third tabs (1702), (1704), (1706), respectively, replaces partial collar (1312). Again, tabs (1702), (1704), (1706) are preferably integral and elastically deformable, although they may also be plastically deformable. Essentially any locking configuration may be utilized by a doctor or surgeon depending upon the desired fit of post (1304).

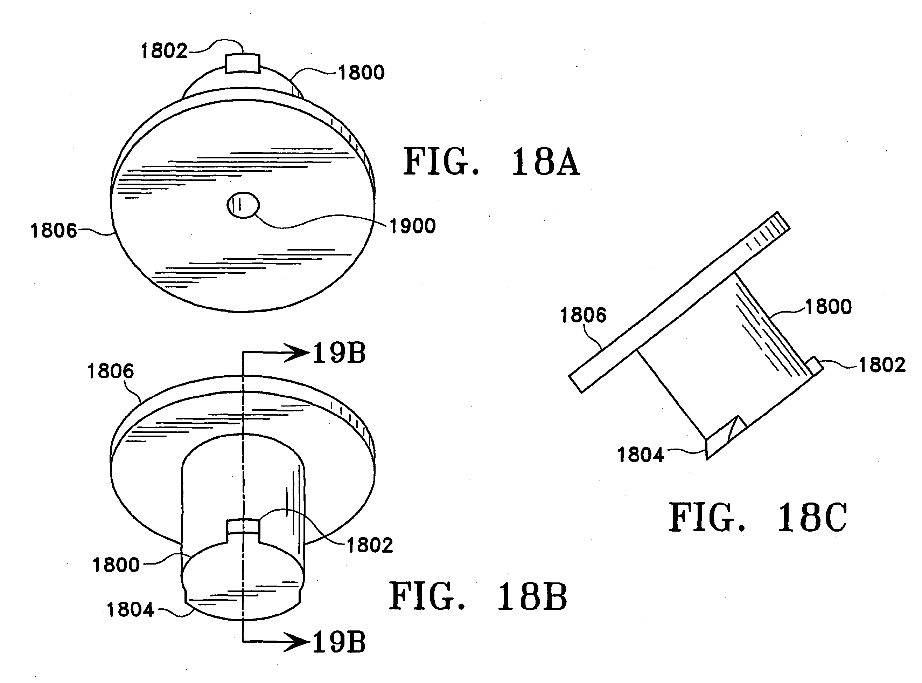

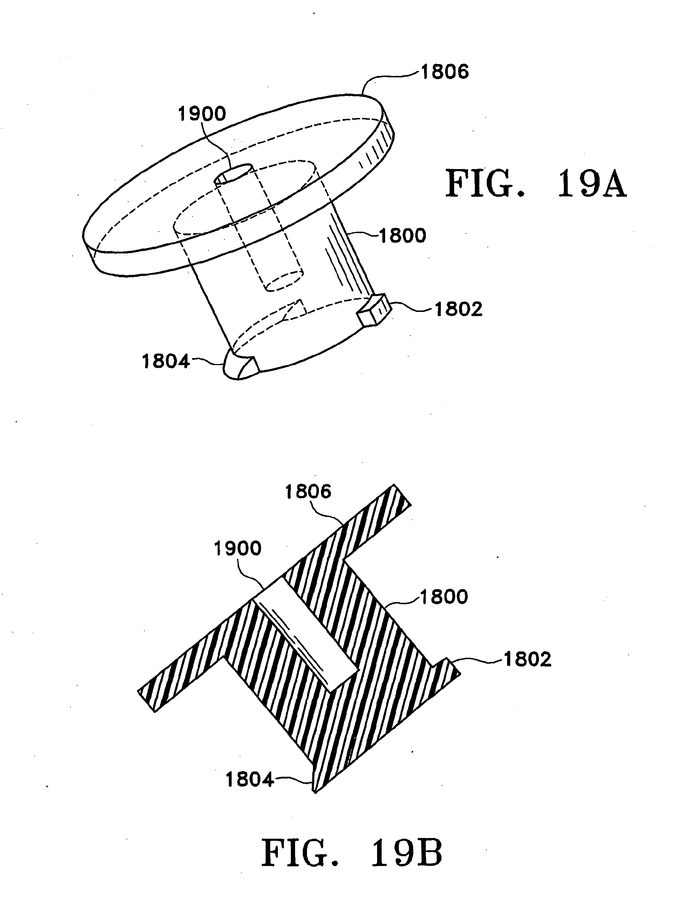

Aside from varying locking mechanisms, the flexibility of the post may be varied as well. As mentioned above, cavities may be disposed within the post to increase the post flexibility. Figure 18A shows a back view of a variation of the cavity from Figure 13B. As seen in Figures 18B and 18C, post (1800) is similar in most respects to the post shown in Figure 13B. Post (1800) is illustrated extending from backing (1806), which is partially shown merely for clarity, with front tab (1802) and partial collar (1804). However, Figure 18A shows a single axial cavity (1900) disposed within and extending from a proximal end of post (1800). Figure 19A shows a perspective view of post (1800) from Figures 18A-18C where axial cavity (1900) is axially disposed within post (1800) and extends partially through. Cavity (1900) may extend through post (1800) perpendicularly to backing (1806) and concentrically along an axis defined by post (1800), but it may also extend off-axis and at an angle, as shown in Figure 13E. Furthermore, cavity (1900) may also extend entirely through post (1800) as a through-hole. Figure 19B shows the cross-section 19B-19B taken from Figure 18B clearly showing cavity (1900) extending partially into post (1800).

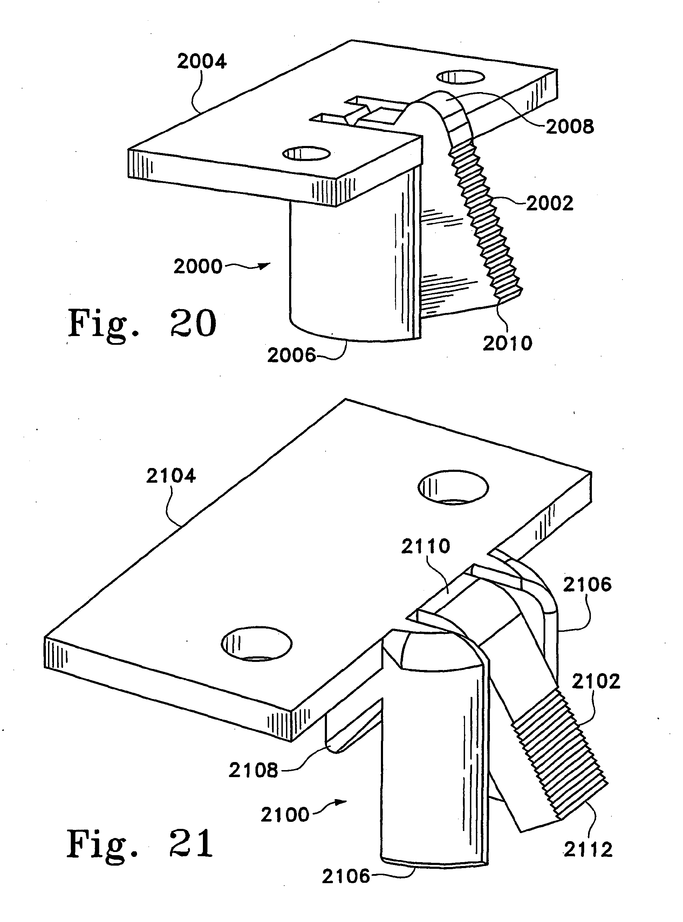

Another variation on the post is shown in Figure 20. Latched post (2000) is shown having beveled latch (2002) pivotally disposed between post members (2006). Latched post (2000) is shown extending from backing (2004) of which only a portion is shown for clarity. Beveled latch (2002) is preferably integrally attached at a proximal end so that latch distal end (2010) is free to move. Beveled latch (2002) is also preferably beveled to provide a gripping surface once the device is secured in the patient. Because latch distal end (2010) may be free to move, latch (2002) may be configured so that latch distal end (2010) may be biased to extend angularly away from post members (2006). As post (2000) is inserted into a patient's cranium, latch distal end (2010) may be urged towards post members (2006) to facilitate insertion by depressing lever (2008), located at the proximal end of latch (2008). Once latched post (2000) has been positioned in the patient, lever (2008) may then be released, thus allowing latch distal end (2010) to protrude angularly against the interior of the hole in the patient's cranium thereby providing a locking action.

A further variation of the post is shown in Figure 21. Here, angled latch post (2100) is preferably an angled latch (2102) having a beveled surface and being integral with backing (2104) of which only a portion is shown for clarity. Angled latch (2102) may be integral with backing (2104) at the latch proximal end (2110) and disposed in-between post members (2106). Angled latch (2102) may further be biased so that the latch distal end (2112) is angled away from backing (2104) and protrudes from in-between post members (2106). Accordingly, as angled latch post (2100) is inserted into the patient's cranium, latch distal end (2112) may similarly be urged towards post members (2106) to likewise facilitate insertion. This movement or urging may be accomplished by depressing latch extension (2108), which may be integrally attached to both backing (2104) and angled latch (2102). Because latch extension (2108) may be attached in apposition to angled latch (2102), depressing it would thereby move latch distal end (2112) accordingly.

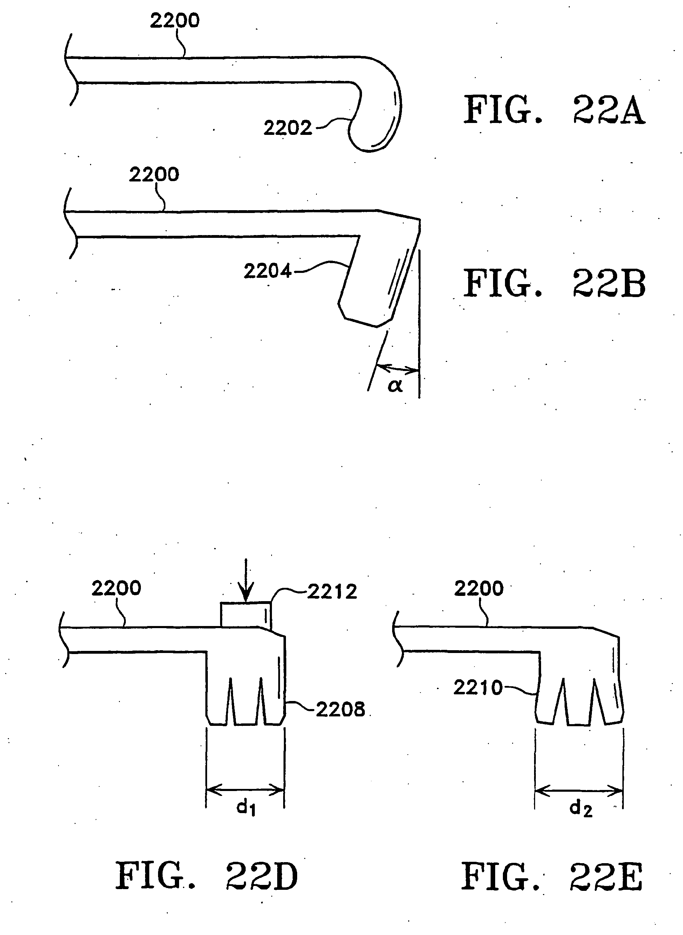

Figures 22A-22B show alternative variations of the post which may include any of the features discussed herein. Figure 22A shows rounded post (2202) having a radiused distal end. Figure 22B shows angled post (2204) which defines a predetermined angle, α, between a plane of backing (2200) and a longitudinal axis defined by angled post (2204).

Figures 22D-22E show an alternative variation where the post comprises radially expandable extensions. Expendable post (2208) is preferably integral with backing (2200) to provide a uniform device. Figure 22D shows expandable post (2208) having a first diameter, d₁. This device may be inserted into the patient's cranium and positioned in a desired location and configuration. Once positioned, the diameter may be expanded by inserting expander device (2212), or using a tool configured to expand radially, which pushes against the inner surfaces of expandable post (2208). The resulting expanded configuration is shown in Figure 22E where expanded post (2210) has a second diameter, d₂, which is larger than first diameter d₁ and thus aids in securing the device in place.

### Variations on Drilled Holes

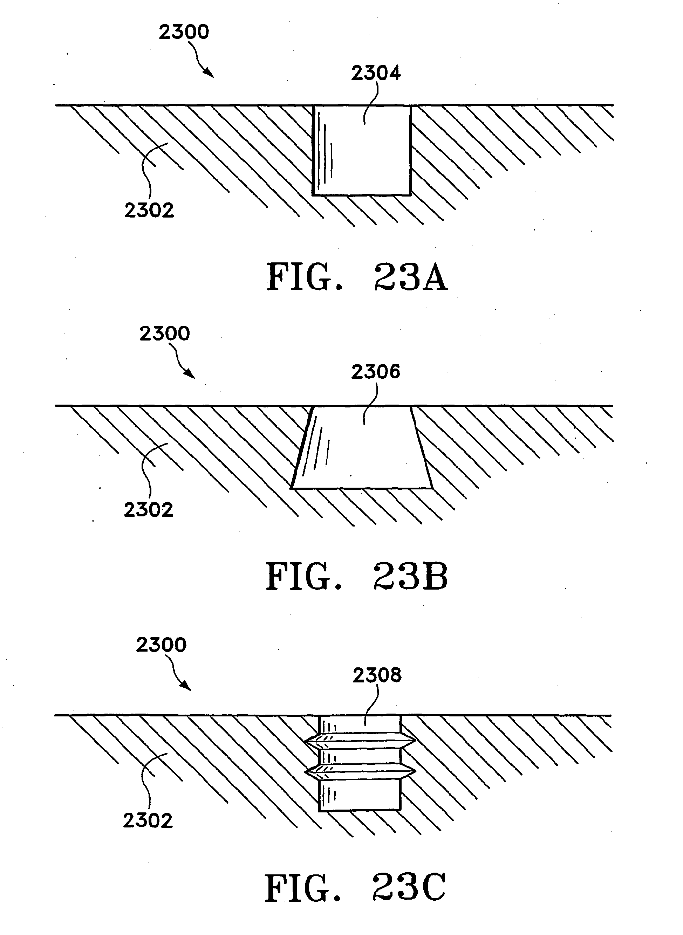

In securing a brow lift device within a patient, a hole may be drilled into the cranium to receive the securing post of the device. As mentioned above, the hole may be drilled by any number of conventional drills or specialized surgical drills. Figure 23A shows a cross-sectional view of a typical drilled hole (2304) in cranium (2300) which extends down into the cranial bone (2302). Figure 23B shows another variation having angled hole (2306) which may be used to receive any of the post variations discussed herein. A further variation is shown in Figure 23C where the hole may comprise keyed hole (2308). This variation shows keyed hole (2308) having two concentric grooves within the hole; however, any number of grooves or variations thereof may be incorporated depending upon the desired hole profile and the tightness of the fit of the post within the hole.

### Variations on Supportive Backings

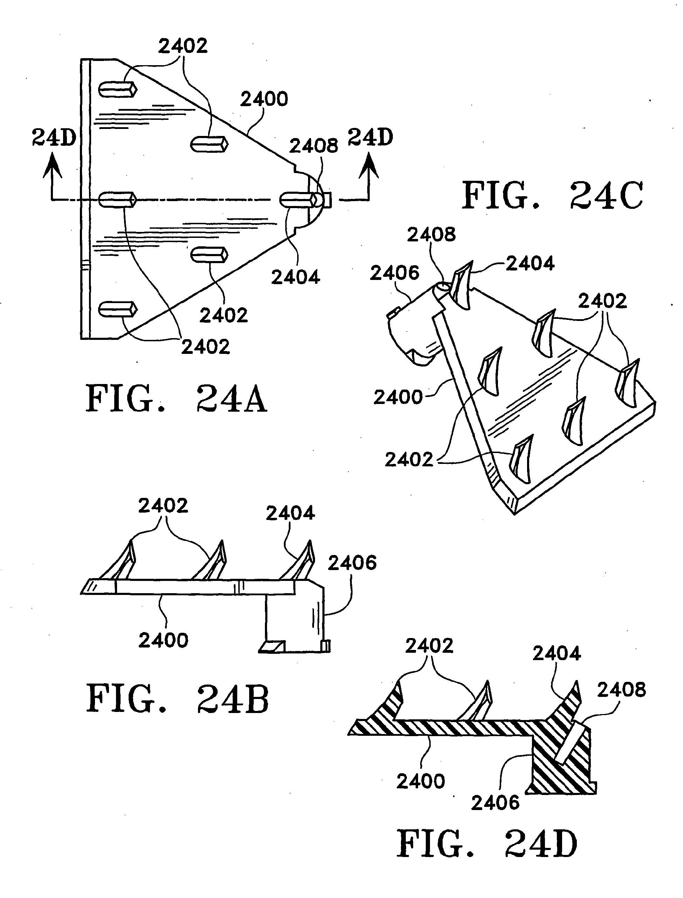

Figures 24A-24D show a second embodiment of device provided with a variation in the brow lift device backing. Figures 24A-24B show a top and side view of a second embodiment of device which is similar in many aspects to the first embodiment of device as shown in Figures 13A-13C. The device comprises supportive backing (2400), post (2406), proximal cavity (2408), and attachment points (2402). However, this second embodiment also comprises an additional leading attachment point (2404). This leading attachment point (2404) may be incorporated as a redundancy to ensure tissue adhesion should the other attachment points (2402) slip or tear from the scalp tissue. Figure 24C shows a perspective view of the device with leading attachment point (2404). And Figure 24D shows a view of cross-section 24D-24D from Figure 24A. Proximal cavity (2408) is clearly seen to extend partially into post (2406); but post (2406) may incorporate other cavities and configurations as discussed above.

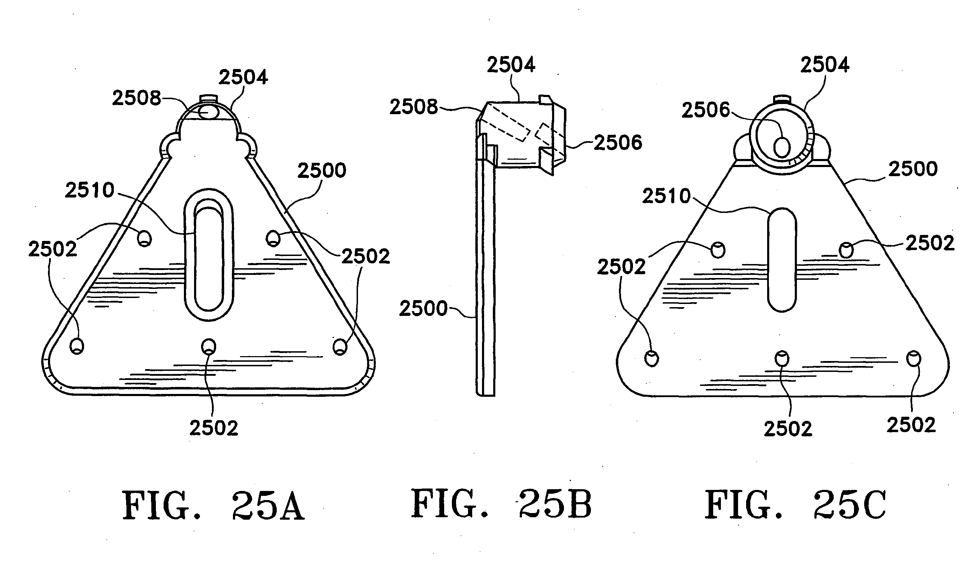

Figures 25A shows a top view of the supportive backing (2500) of a third embodiment of device. This third embodiment is also similar in many aspects to the first embodiment of device shown in Figures 13A-13C. The device may comprise post (2504), proximal cavity (2508), and through-hole (2510), which may be slotted or may comprise any other shape. Also, as seen in Figures 25B and 25C, the device may also comprise distal cavity (2506); however, this third embodiment may have separatable attachment points which may be held in attachment point locations (2502). This third embodiment may allow a doctor or surgeon to attach variously shaped attachment points in a variety of orientations relative to one another depending upon the desired result Moreover, this third embodiment may allow one to selectively attach attachment points at desired attachment point locations (2502). Any number of attachments points may be utilized; however, it is preferable that at least three attachment points or tines spaced relatively apart be used to optimize the holding capacity of the device to the tissue.

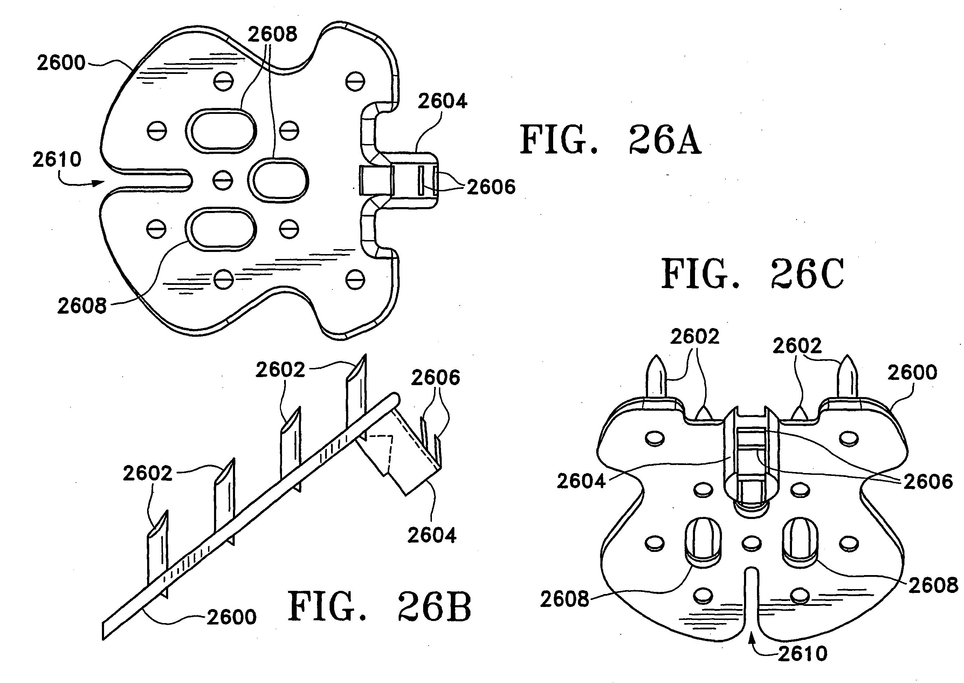

Figure 26A shows a top view of a fourth embodiment of device comprising an alternative variation for supportive backing (2600) which is configured to be flexible and hold multiple attachment points (2602). This fourth embodiment may be configured to reduce the amount of material used and simultaneously increase the flexibility to allow backing (2600) to conform to the patient's cranium. Flexibility may be achieved via the use of through-holes (2608) and slot (2610) which are seen in Figures 26A and 26C. This fourth embodiment also may incorporate post (2604) which may comprise anchoring tabs (2606), as seen in the side view of Figure 26B, to aid in securing the device to the cranium.

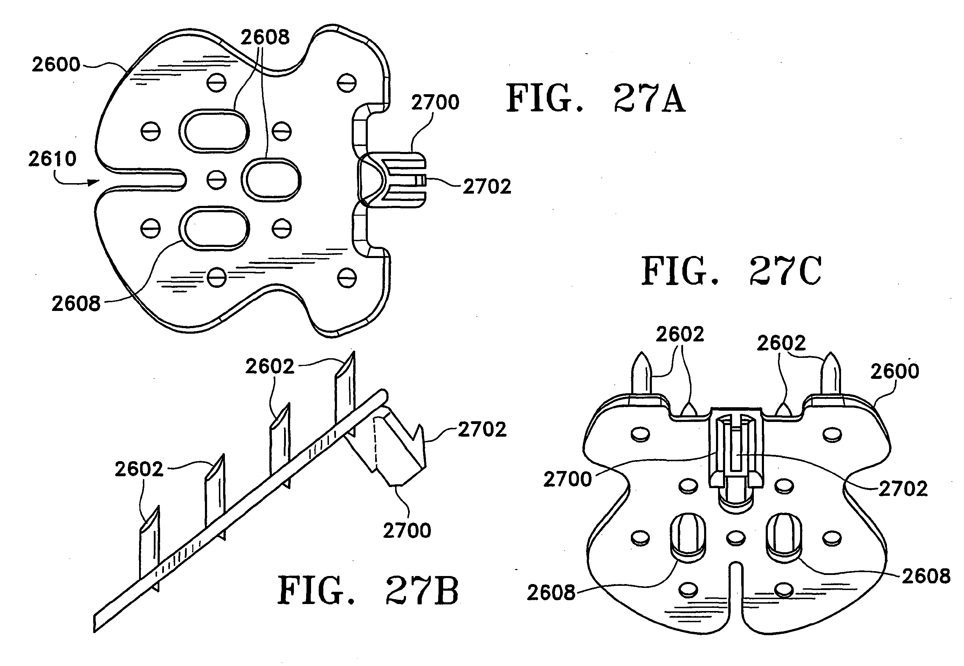

Figure 27A shows a top view of a fifth embodiment of device comprising an alternative variation for supportive backing (2600) which is similar in most aspects to the device shown in Figure 26A. As seen in Figures 27A-27C, particularly 27B, the fifth embodiment incorporates latched post (2700). Post (2700) may utilize a latching mechanism similar to the latched posts illustrated in Figures 20-21. This particular post comprises latch (2702) which is shown as having a hooked distal end.

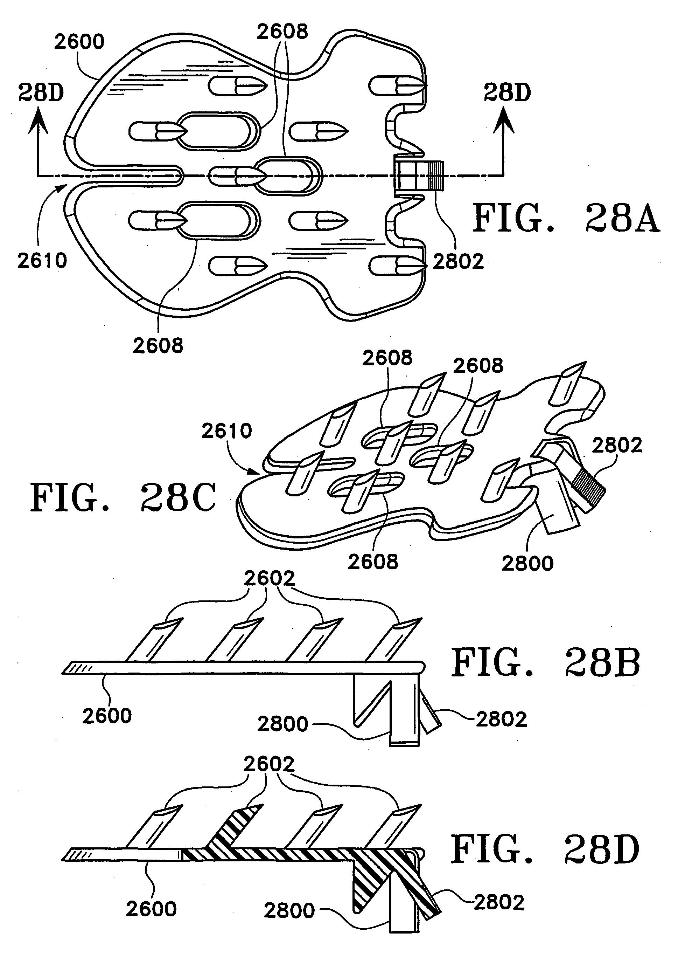

Figures 28A-28C shows top, side, and perspective views of a further variation for supportive backing (2600) in a sixth embodiment of device. This variation illustrates latched post (2800) having beveled latch (2802) which may be similar to the latching device shown in Figure 21. Figure 28D shows a view of cross-section 28D-28D taken from Figure 28A. The latched post (2800) and the configuration of latch (2800) may be seen where latch (2802) is preferably integral with backing (2600).

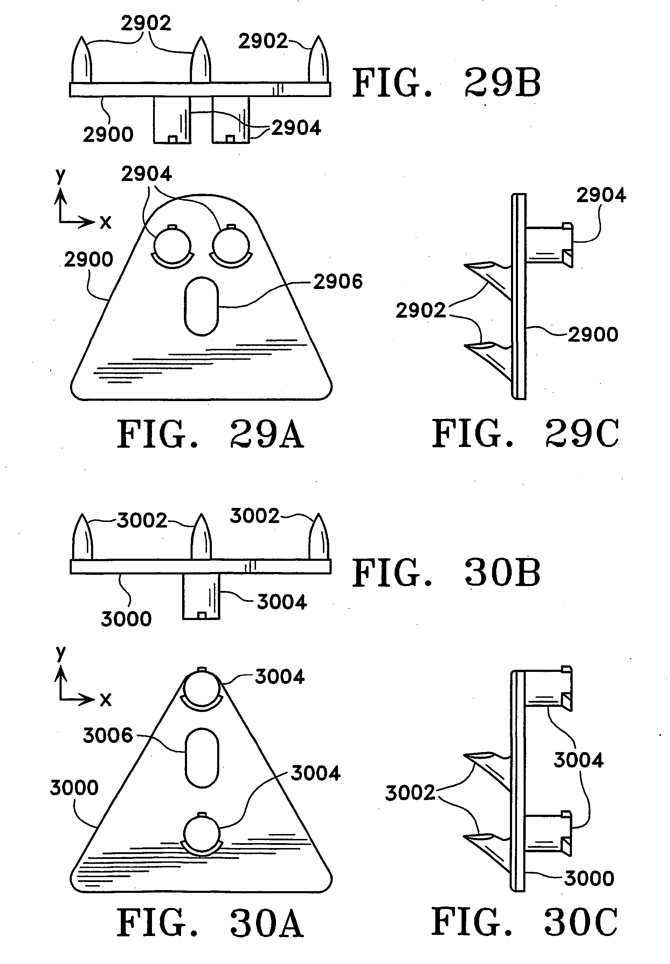

In addition to alternative backings, variations of MTDS devices having multiple posts may also be utilized. Figure 29C shows a seventh embodiment of device also having attachment points (2902) and through-hole (2906). As seen further in Figures 29B, this seventh embodiment may comprise a configuration where two posts (2904) are attached to backing (2900). Posts (2904) are preferably attached integrally to backing (2900) and may be orientated, as seen in Figure 29A, such that posts (2904) are aligned along an x-axis. The addition of a second post along the x-axis may aid in increasing the device resistance to rotation about the posts (2904) once it is inserted into the cranium. This added rotational stability may then allow the device to be inserted at various angles within the cranium relative to the tissue to be lifted depending upon the desired results.

A further alternative backing having multiple posts is shown in the eighth embodiment of device in Figure 30A. Also seen in this eighth embodiment are attachment points (3002) attached to backing (3000) and through-hole (3006) defined within backing (3000). However, this eighth embodiment comprises two posts (3004), which are preferably integral with backing (3000), aligned along a y-axis. The additional post along the y-axis may aid greatly in also increasing the device resistance to rotation about posts (2904). This eighth embodiment likewise may allow the device to be inserted at various angles within the cranium depending upon the desired results and the angle of desired lift. Furthermore, this particular variation may be desirable where cranial physiology would prevent two adjacent posts from being secured into the cranium.

### Placement Tools

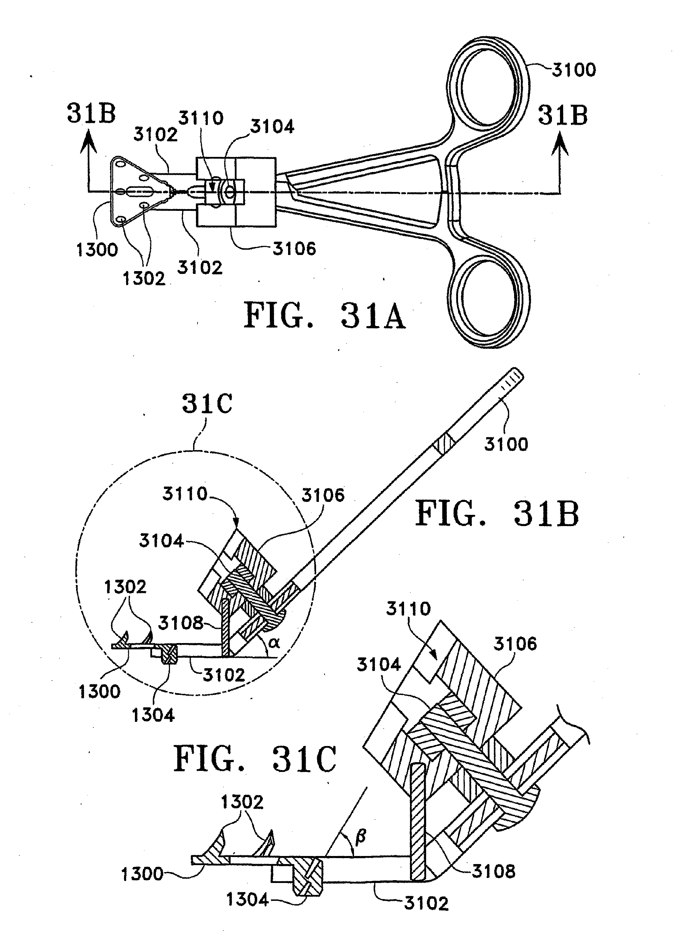

Many of the variations on the brow lift device may be inserted and secured into a patient in a number of ways. One such method involves using an insertion tool of a type shown in Figure 31A. This variation shows a top view of such a tool which may serve several functions. This tool comprises manipulation handle (3100), by which a doctor or surgeon manipulates, for example, the device of Figures 13A-13C. As shown further in Figure 31B, cross-section 31B-31B from Figure 31A, handle (3100) may be hinged by any conventional methods but shown here as bolt hinge (3104). At a distal end of handle (3100) are grasping members (3102). These grasping members (3102) may generally be designed to have opposing members which may be urged together or apart, i.e., to close or open, as handle (3100) is urged about hinge (3104).

To prevent uncontrolled rotation of handle (3100) and to provide a way of securely grasping the device, handle (3100) may also comprise a locking mechanism which may hold handle (3100) and grasping members (3102) in a desired position. Grasping members (3102) are preferably designed or configured to securely hold the supportive backing (1300) relatively planar with grasping members (3102) such that attachment points (1302) face away from the patient during insertion. It is further preferable that grasping members (3102) securely hold the MTDS device via anchoring post (1304) to allow easy handling and insertion. As seen in Figure 31B, grasping members (3102) are preferably angled relative to a plane defined by handle (3100) at a predetermined angle, α, to further allow easy insertion of the device.

Figure 31C shows a close-up cross-sectional view of the distal end of the insertion tool. As shown, also attached to hinge (3104) is support block (3106). Support block (3106) is preferably configured to attach to handle (3100) at hinge (3104) yet still allow rotational movement of the tool about hinge (3104). Support block (3106) also preferably defines channel (3110) through a top surface of support block (3106), as shown in Figures 31A-31C. Channel (3110) may run substantially parallel relative to a symmetrical axis defined by the insertion tool. Support block (3106) may be supported by support post (3108) which may help in preventing rotation of support block (3106) about hinge (3104) as well as maintaining a position of the block relative to handle (3100).

Further seen in Figure 31C, channel (3110) in support block (3106) is preferably angled relative to the plane defined by handle (3100). While grasping members (3102) are angled at an angle, α, relative to handle (3100), channel (3110) may be angled relative to grasping members (3102) at a desired angle, β. This angle β is preferably similar to the angle formed by attachment points (1302) relative to supportive backing (1300). Angling channel (3110) may allow a mating block, described below in further detail, to run along channel (3110) and press against the tissue to be lifted against attachment points (1302). A block pressing against tissue to be set on attachment points (1302) allows for optimal piercing of the tissue if the force applied by the block is in the same or similar angle or direction as attachment points (1302).

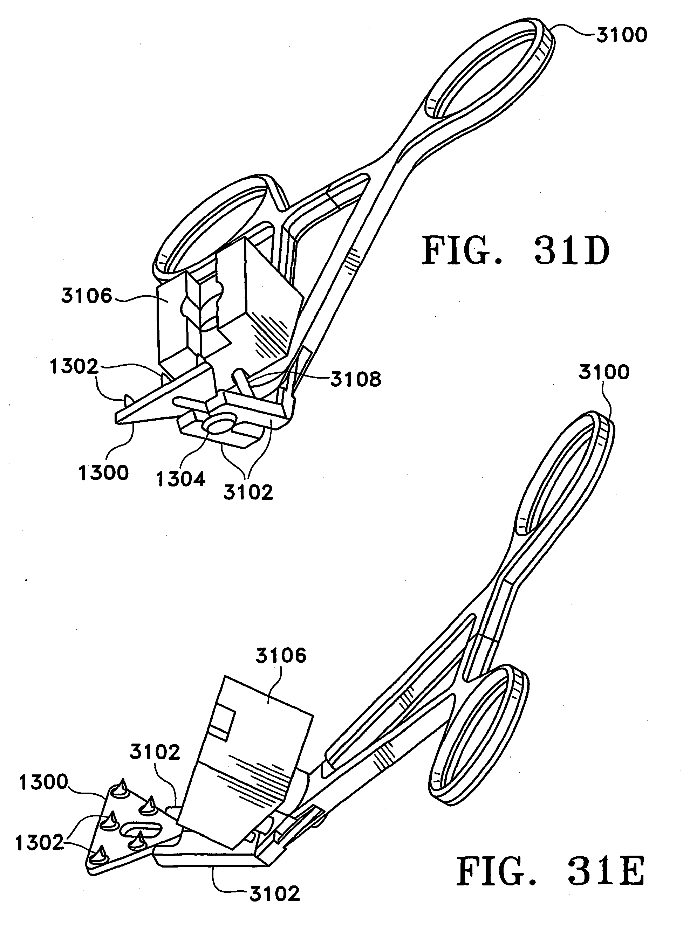

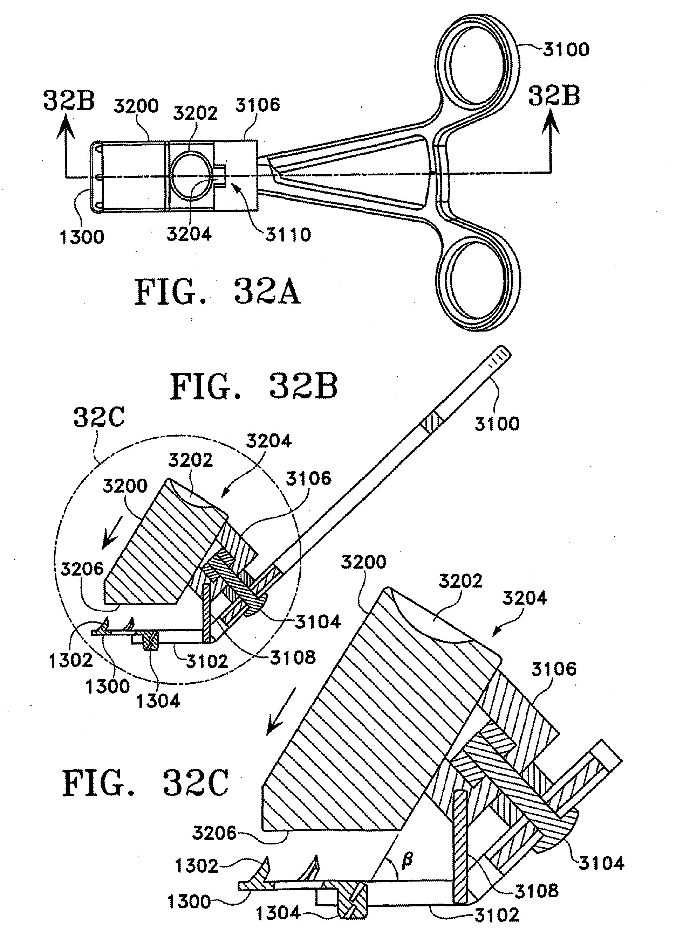

Figures 31D and 31E show a bottom and a top perspective view, respectively, of the insertion tool from Figure 31A grasping an device. As seen in Figure 32A, the same insertion tool from Figure 31A is shown with the addition of depressible block (3200) mated with support block (3106). Depressible block (3200) may be mated with support block (3106) via channel (3110), into which mating slide (3204) may be inserted. Slide (3204) may be an integral extension of depressible block (3200) and is preferably configured to allow a degree of tolerance relative to channel (3110) so that depressible block (3200) may slide freely or when urged via channel (3110) and mating slide (3204), as shown by the arrow in Figure 32B.

Figure 32B also shows a cross-section 32B-32B from Figure 32A. Depressible block (3200) further illustrates depression region (3202), which may be a slight indentation defined in the surface facing away from the patient during insertion. Depression region (3202) may serve as a locator for the optimal region the physician may depress to force depressible block (3200) and contact surface (3206) downward against the tissue and attachment points (1302) in order to set, or pierce, the tissue. Figure 32C shows a close-up cross-sectional view of the distal end of the insertion tool with depression block (3200) inserted. Contact surface (3206) is the surface which ultimately presses the tissue against attachment points (1302) and is preferably relatively parallel with the plane defined by grasping members (3102) and supportive backing (1300) to present the greatest surface area pressing against the tissue. Depressible block (3200) is further preferably configured to slide or run along the same angle, β, at which support block (3106) is set to provide a planar contact surface (3206) to press against the tissue at an optimal angle, which may be at the same or similar angle as Attachment points (1302), as discussed above.

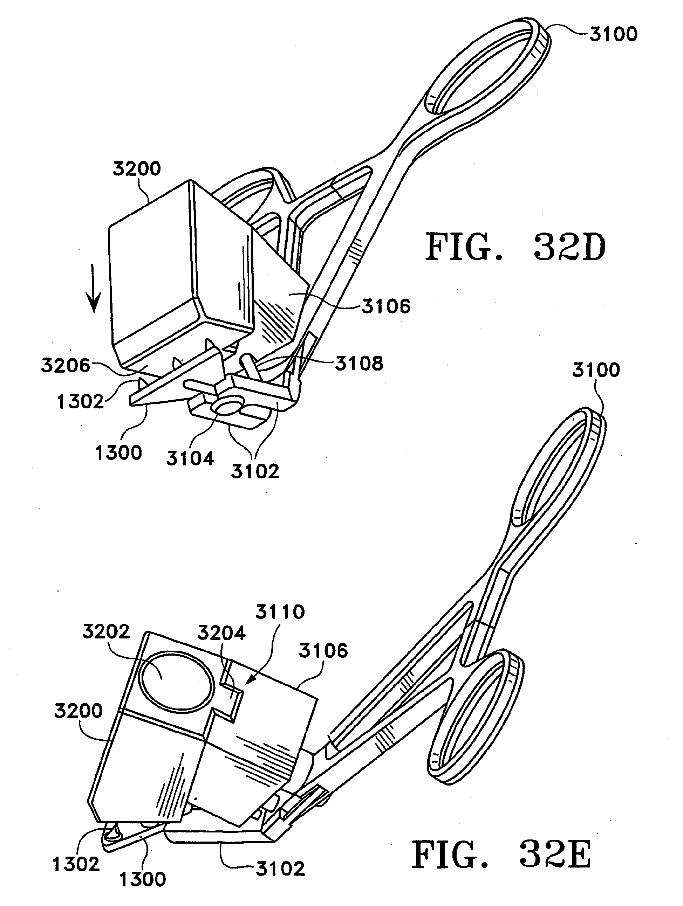

Figures 32D and 32E show a bottom and a top perspective view, respectively, of the insertion tool from Figure 32A with depressible block (3200) set in channel (3110). Although the placement tool has been described with depressible block (3200), the tool may also be used without a block for depressing the tissue or scalp against the attachment points (1302). Rather, affixing or setting the tissue may also be done by hand, i.e., simply depressing the tissue with the hand and fingers against attachment points (1302).

### Orbital Fracture Procedures

Another variation of the present invention includes approximation of soft tissue in orbital fracture repair and other craniofacial and maxillofacial surgical procedures. The supportive backing or plate set fragmented bones. The present invention also includes a plurality of attachment points which extend from the supportive backing such that soft tissue may be conveniently suspended on the attachment points. Examples of attachment points include tines.

Notably, the present invention eliminates the use of sutures to fixate soft tissue to the underlying fracture site. Consequently, typical problems associated with suturing soft tissue to the underlying bone are eliminated.

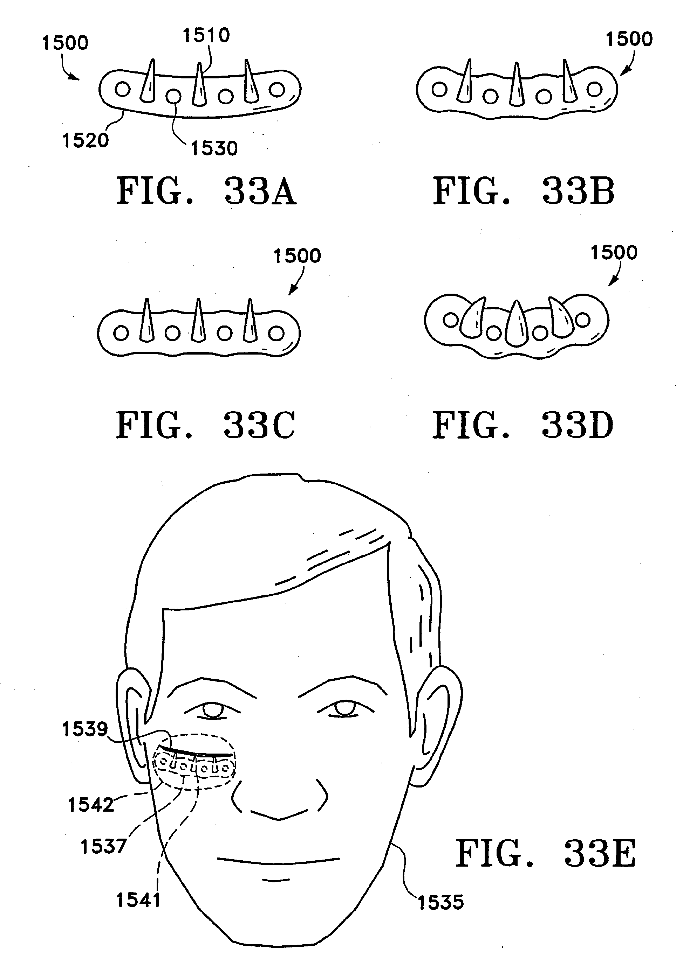

The present invention includes various shapes which are useful in approximation of soft tissue in orbital fracture repair and other craniofacial and maxillofacial surgical procedures. A set of shapes not in accordance with the invention is illustrated in Figures 33A to 33D. Figures 33A to 33D are front views of a tissue approximation device (1500) not in accordance with the present invention and suitable for use in orbital fracture reconstruction procedures. As shown in Figure 33A, attachment points (1510) extend from backing (1520).

The tissue approximation device (1500) also features a number of through-holes (1530). The through-holes provide an opening for receiving a fastener such as a pin or screw. The holes (1530) may be equally spaced or unequally spaced along the backing (1520). There may be one or more holes (1530).

In addition to the shapes shown in Figures 33A-33D, the plate or supportive backing may be shaped as a character such as but not limited to C, H, I, L, T, U, V, Λ, and ∩. The supportive backing may also be curved away from the direction of the tines or curved in a direction orthogonal to the direction of the tines. The supportive backing may also be convex or concave when viewed from the front or the side (not shown).

Except where stated otherwise, the characteristics of the attachment points (1510) and supportive backing (1520) are similar to the attachment points and backings described in the variations set forth above. For example, the supportive backing is preferably fabricated from biocompatible materials, biodegradable materials, or materials which are generally absorbable by the patient. The device may also be made from biological materials.

The device may further contain bioactive compounds or therapeutic agents. Such agents may be impregnated in the device, coated on the device, sprayed, or otherwise deposited on the device. Multiple coatings may be applied to delay release of such agents. Suitable agents include proteins, pharmaceuticals, genetic material, and other chemicals or compounds which have a useful effect in humans. Other non-limiting examples of agents include hydroxyapatites, tricalcium phosphates, bone growth factors, and bone morphogenic proteins.

The device may also be made of a material and thickness such that it may be shaped intra-operatively to the patient's anatomy by applying heat to the device. Such devices are well suited for orbital reconstruction and suspensions where curves are desirable to accommodate facial bones.

An illustration of the use of a device not in accordance with the present invention is shown in Figure 33E. Figure 33E shows a head (1535) with a tissue approximation device (1537) secured to an orbitalfacial fracture site (1542) underneath wound (1539). The device (1537) is shown as a rigid plate and is useful in setting fragmented bones.

Characteristics of the supportive backing of device (1537) will depend on its application. In this illustration, where bone setting is required, the backing must be generally rigid and have a sufficient thickness to fasten the bone fragments together. In other applications, however, where the device is used for tissue approximation and no bone setting is required, the backing may be less rigid, less thick, and more conforming.

Figure 33E also shows the lower portion of wound (1539) set or suspended on tines (1541). In this manner, the soft tissue covering the device (1537) remains suspended and there is no need for additional sutures to attach the soft tissue to the underlying bone. There is also no need for any additional steps to suspend the soft tissue.

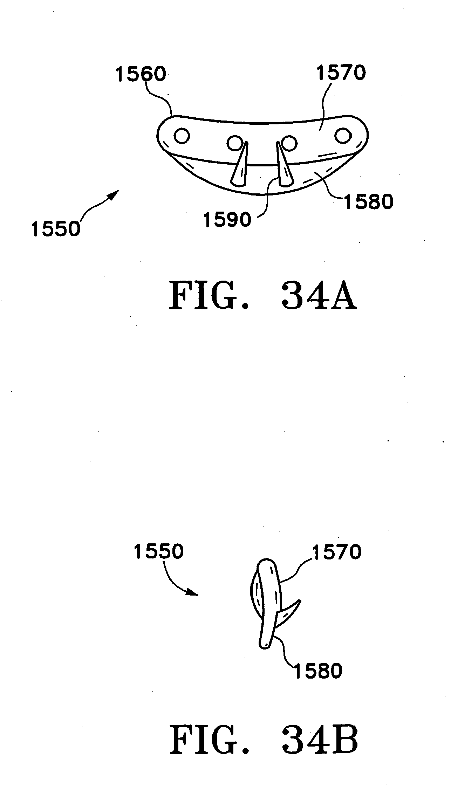

Figures 34A and 34B show a further variation also not in accordance with the present invention which is also useful in orbital reconstruction procedures. In particular, Figure 34A shows a tissue approximation device (1550) having a supportive backing (1560) divided into two discrete regions. The first or plate region (1570) includes several through-holes and is "tineless." That is, no tines or attachment points are shown in the plate region (1570) of Figure 34A. The second or tine region (1580) features two tines (1590) to serve the function as indicated in the above described variations. The plate region and tine region may be separate structures joined together or they may be integral with one another.

Figure 34B shows a side view of the tissue approximation device having a variation in thickness. In particular, the tine region (1580) is thinner than the plate region (1570). Such a device is suitable for applications requiring a thicker substrate in one bone location. In orbital and maxilla fractures, for example, devices with a varying thickness can be useful. Of course, the invention is not limited to the particular variation shown in Figures 34A and 34B. For example, the tine region may be thicker than the plate region (not shown).

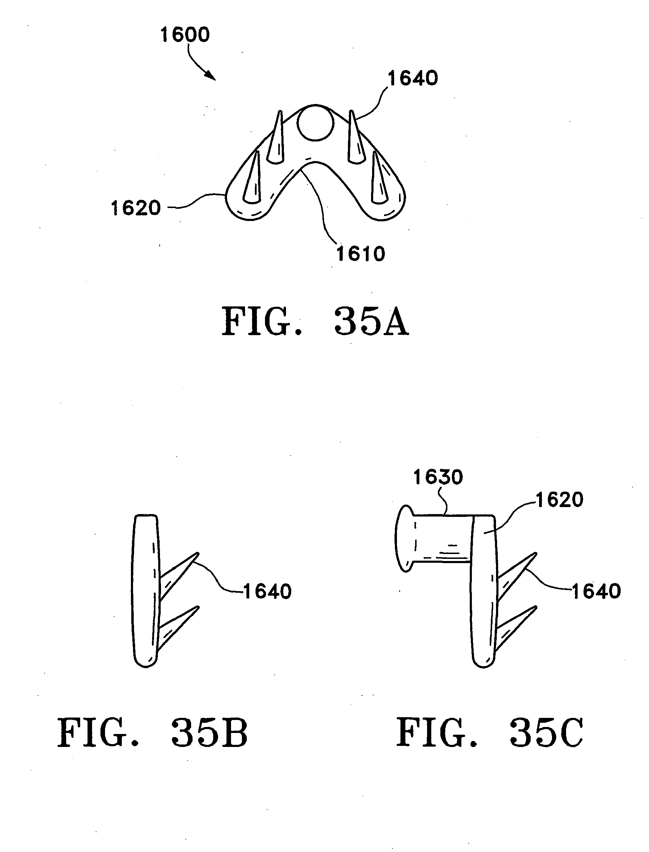

Figure 35A and 35B illustrate another device not in accordance with the present invention. In particular, Figure 35A shows a tissue approximation or MTDS device (1600) in a horseshoe shape. Arc (1610) is provided to avoid covering nerves such as the infraorbital nerve in, for example, midface lift procedures. While a horseshoe shape is shown in Figure 35A, the device may include other shapes having arcs, slots, or curves which avoid covering nerves or other anatomical structures which are desirably left uncovered.

In the modification of the Figure 35A + 35B device shown in Figure 35C, backing (1620) has an auchoring post (1630) extending therefrom to secure the device in bone. The anchoring post (1630) may eliminate the need for separate fasteners. Figures 35A to 35C also feature four tines symmetrically disposed on backing (1620). The tines serve the same function as described in the preceding variations of the present invention.

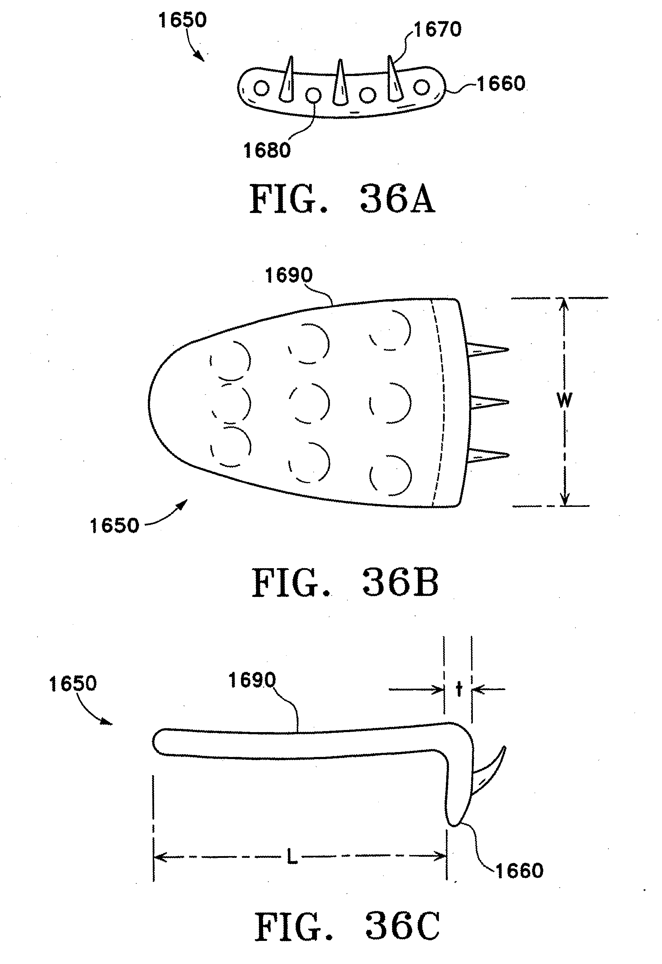

Figures 36A to 36C illustrate a tenth embodiment of the present invention useful in orbital rim and orbital floor reconstruction. As shown in Figures 36A to 36C, the device (1650) includes a backing (1660), tines (1670), and through-holes (1680) similar to the variations described above. However, device (1650) features a floor (1690) perpendicularly extending from backing (1660). The floor (1690) is shown as substantially flat and has a width approximately equal to the width of the plate or backing (1660). Preferably, the width W of the backing (1660) is sized equal to or less than the width of the orbit The thickness t of backing (1660) is preferably in the range of 0.1 to 5 mm and more preferably between 0.5 to 2.5 mm. The length L of the floor (1690) is limited also by the depth of the orbit and the thickness of the floor is preferably in the range of 0.3 to 1 mm.

The device shown in Figures 36A to 36C is particularly suitable in severe orbital fractures that includes fractures of the orbital floor where additional support is required. That is to say, a floor (1690) is suitable in repairing severely damaged sites where the orbit bones are fragmented and fixation is needed in multiple dimensions. The floor is shown having a particular shape however the invention is not so limited. The floor may have other shapes and may be adapted to particular sites and depths as appropriate for the severity and type of fracture.

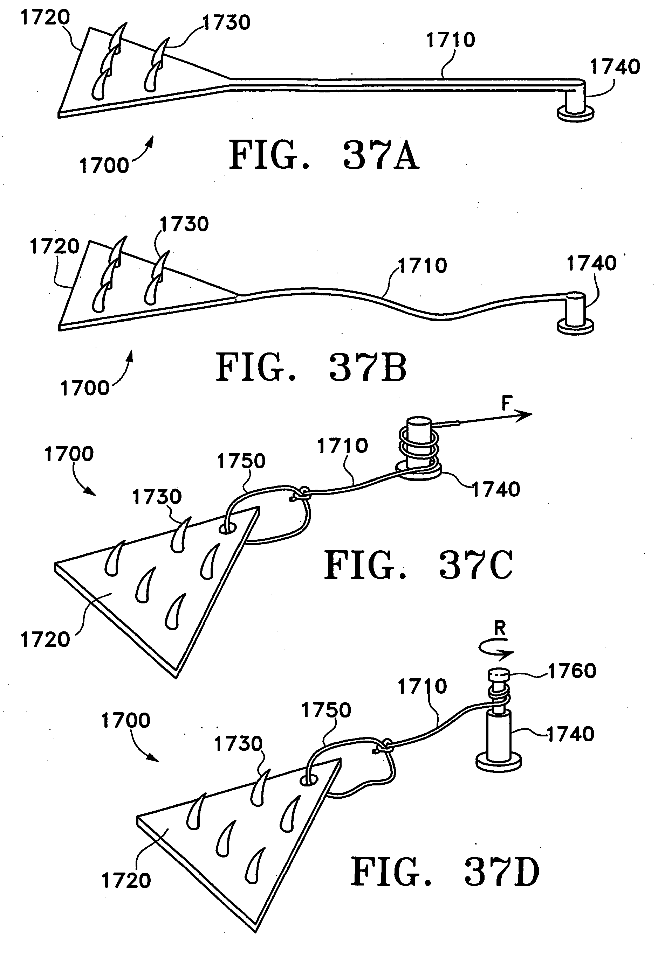

Figures 37A to 37B illustrate an eleventh embodiment of the present invention. Figures 37C and 37D show devices not in accordance with the invention. In all of Figures 37A to 37D a tissue approximation or MTDS device (1700) is shown with an extension member (1710) extending from supportive backing (1720), is shown. Similar to the variations described above, backing (1720) includes one or more tines (1730) for suspending soft tissue such as cheek tissue in the orbital region. Unlike the previous variations, however, an anchor post (1740) is separated from backing (1720) by extension member (1710).

This makes the device is suitable for procedures where the preferred anchoring position is not adjacent the soft tissue to be suspended. The present invention thus provides for the suspension of soft tissue remote or distal to an anchoring position. While only one plate is shown attached to anchor (1740), the present invention also encompasses multiple plates attached to a single post.

Extension member (1710) may be either solid or flexible (such as a tether) as shown in Figures 37A and 37B respectively. If solid, a surgeon may be provided with a number of devices having varying lengths. A device having a solid extension member may be used to indirectly suspend soft tissue above or below the anchor post (1740).

Soft or threadlike extension members may be suitable for indirectly suspending soft tissue below the anchoring position. Advantageously, the length of soft or threadlike extension members may be adjusted and varied during a surgical procedure.

For example, Figure 37C shows a flexible extension member (1710) being manipulated by a force F which decreases the distance between the backing (1720) and post (1740). In this manner, a post may be secured to a bone site and the backing or plate (1720) may be positioned a selected distance from the anchor (1740).

Another variation is shown in Figure 37D. In Figure 37D, the extension member (1710) is adjusted by rotating a knob (1760) to wind the extension member around the knob thereby decreasing the distance between the post (1740) and the plate or backing (1720).

The extension member may be joined to the plate or backing (1720) in a number of ways including a suture (1750), adhesive, a knot, an ultrasonic weld, a pressure fit, or any other suitable jointing technique which is in accordance with the present invention.

### Fracture Fixation Fasteners and Spacers

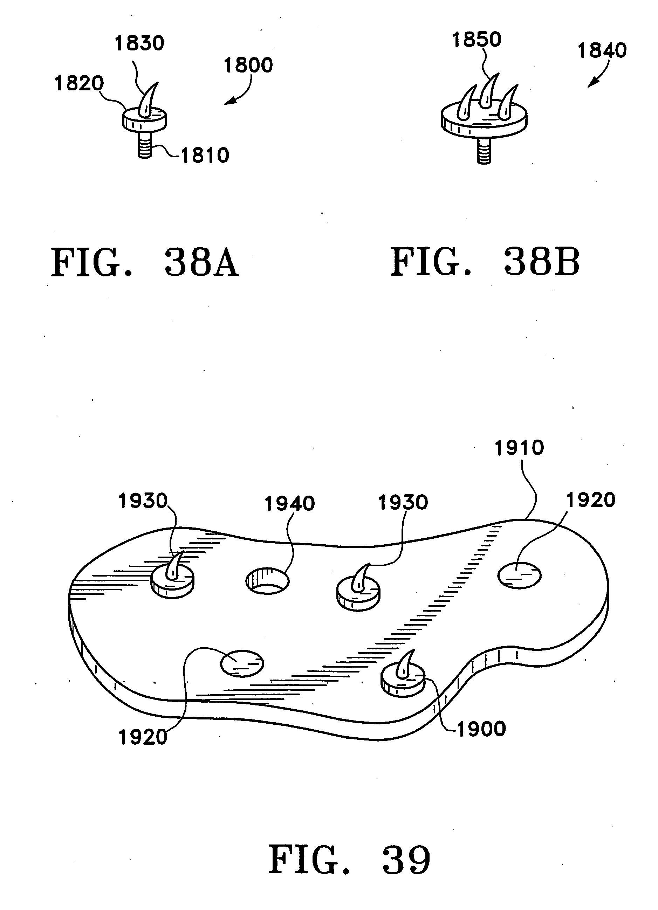

A twelth embodiment of device in accordance with the present invention is illustrated in Figures 38A and 38B. In particular, Figure 38A shows fastener (1800) in the shape of a screw. Fastener (1800) includes a body (1810) and an end or head (1820). Extending from head (1820) is an attachment point or tine (1830). Tine (1830) terminates in a sharp point and is adapted to fix or suspend tissue. Figure 38B shows fastener (1840) having a plurality of tines (1850).

The device illustrated in Figures 38A and 38B is suitable for fixing or suspending tissue to the bones of the cranium or face as well as other bones or tissue throughout the body where suspension of soft tissue is desired and preferably where the soft tissue is adjacent to the bone site. A single fastener may be used in accordance with the present invention to suspend soft tissue or a number of fasteners may be selectively deployed into bone sites. The fasteners of the present invention may therefore be used alone or in combination with other devices.

While fastener (1800) is shown as a screw with threads, the invention is not so limited. Fastener may be a pin or wire or other shape which can be inserted into a hole in a bone and secured thereto. Other suitable fasteners in accordance with the present invention include bone anchors, tacks, and rivets having at least one tine or attachment point extending from its head or proximal end. Furthermore, the fastener may be either self tapping or not self tapping. A self tapping screw may, for example, have a V-thread and terminate in a sharp tip (not shown) whereas a non-self tapping screw may be buttress-threaded with a rounded tip.

Non-limiting examples of suitable materials for fasteners include stainless steel, Ti alloy, CoCr alloys, polylactic acid or polyglycolic acid, and Nylon. Other suitable materials include those biocompatible and bioabsorbable materials previously referenced in connection with other variations of this invention and composites thereof.

Figure 39 illustrates an application of a device not in accordance with the present invention employing the fasteners (1900) of Figure 38A in combination with a plate (1910) and additional "tineless" fixation screws (1920). Accordingly, a relatively large fracture fixation plate (1910) may be secured to various bone fragments (not shown) with selected regions having attachment points or tines (1930) extending therefrom. The surgeon thus has control and flexibility in determining where soft tissue shall be suspended. Figure 39 also shows one empty through-hole 1940. Through-holes may be filled with a fastener or even a therapeutic agent depending on the application.

While the backing (1910) is shown as relatively large, the backing may be variously sized. For example, the backing may be shaped as a ring or washer having a single though-hole to receive a fastener.

The backing or plate (1910) may also include one or more discrete attachment regions (not shown) having, for example, tines. A number of fasteners (1900) having tines (1930) may be positioned in selected though-holes to supplement suspension of the soft tissue or to further distribute the tension forces within suspended tissue.

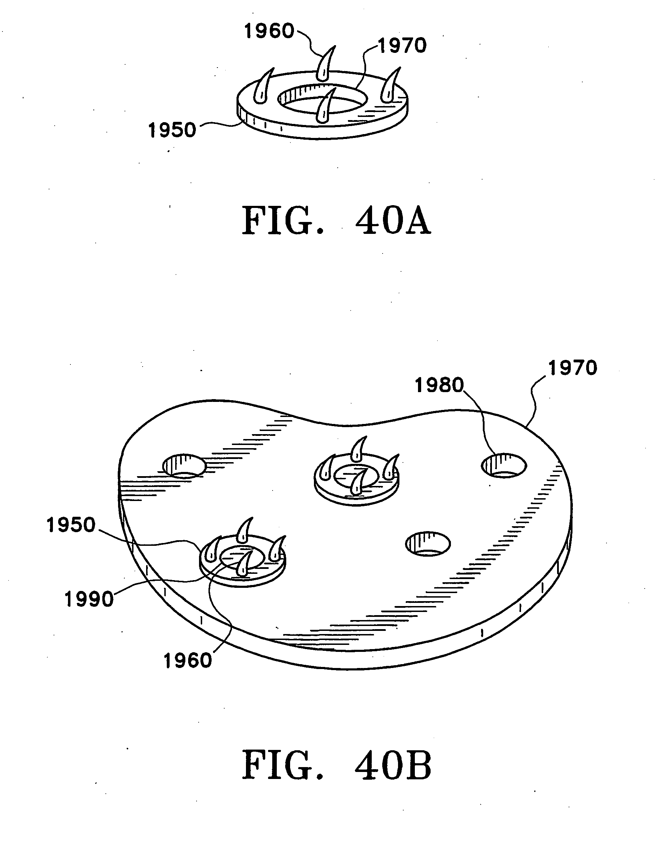

Figures 40A and 40B show another variation not in accordance with the present invention. In Figure 40A, spacer (1950) features four tines (1960). Spacer (1950) may be used alone or in combination with other devices to suspend tissue. The spacer (1950) shown in Figures 40A and 40B includes an aperture (1970) for receiving a fastener. However, a spacer need not have an aperture or the aperture may have other shapes (not shown).

Another configuration is shown in Figure 40B. In Figure 40B, spacer (1950) is used in combination with a relatively large fracture fixation plate (1970) which has through-holes (1980). Tineless fasteners (1990) may be used to secure the plate (1970) in combination with spacers (1950) to an underlying bone site. As shown in Figures 40B, the spacer is secured on top of the plate with its tines extending therefrom. The tines are attachment points for suspending adjacent soft tissue and are designed in accordance with the variations described above.

Further, the present invention is not limited to bone fracture repair sites. The present invention may also be used in applications where no bone fractures are present or where bone fractures have healed. For example, the device may be attached to a healthy bone site to cure or compensate for sagging tissue. Moreover, the device of the present invention may be used to supplement previous surgeries in which the soft tissue was elevated from the underlying bone and now needs re-anchoring.

We have described this invention by example and by description of the physical attributes and benefits of the structure. This manner of describing the invention should not, however, be taken as limiting the scope of the invention in any way.

## Claims

1. An implantable tissue approximation device comprising:
(a) a supportive backing;
(b) a plurality of attachment points extending from said backing; and
(c) a bone anchoring region integral with said backing, wherein:
the supportive backing (1300, 1806, 2104, 2200, 2400, 2500, 2600, 2900, 3000, 1620, 1660, 1720) has opposing front and back sides;
the plurality of attachment points (1302, 1600, 2402, 2602, 2902, 3002, 1640, 1670, 1730, 1850) each extend from said backing in a canted manner; and
said bone anchoring region defines a post (1304, 1630, 1690, 1740, 1800, 2000, 2100, 2202, 2204, 2208, 2210, 2406, 2504, 2604, 2700, 2800, 2904, 3004) extending from said backing and constructed and arranged to secure the device to bone.

2. The tissue approximation device of claim 1 wherein said supportive backing (1300, 1806, 2104, 2200, 2400, 2500, 2600, 2900, 3000, 1620, 1660, 1720) comprises at least one discrete contact area and said plurality of attachment points (1302, 1600, 2402, 2602, 2902, 3002, 1640, 1670, 1730, 1850) are situated in said at least one discrete contact area.

3. The tissue approximation device of claim 1 wherein said attachment points (1302, 1600, 2402, 2602, 2902, 3002, 1640, 1670, 1730, 1850) have more than one shape.

4. The tissue approximation device of claim 1 wherein said device also comprises a center and said attachment points (1302, 1600, 2402, 2602, 2902, 3002, 1640, 1670, 1730, 1850) are progressively shorter the closer they are to the center of said device.

5. The tissue approximation device of claim 1 wherein said attachment points (1302, 1600, 2402, 2602, 2902, 3002, 1640, 1670, 1730, 1850) are varied in orientation on said backing (1300, 1806, 2104, 2200, 2400, 2500, 2600, 2900, 3000, 1620, 1660, 1720).

6. The tissue approximation device of claim 1 wherein said backing (1300, 1806, 2104, 2200, 2400, 2500, 2600, 2900, 3000, 1620, 1660, 1720) comprises a plurality of openings (2502) through which attachment points (400) are placed.

7. The tissue approximation device of claim 6 wherein said attachment points (400) are secured to said backing (2500) by a flange (404) or hub.

8. The tissue approximation device of claim 1 comprising a first end area having extending attachment points (1302, 1600, 2402, 2602, 2902, 3002, 1640, 1670, 1730, 1850) and an opposing second end area having one or more openings through said backing.

9. The tissue approximation device of claim 8 wherein said first end area attachment points are canted toward said second end area.

10. The tissue approximation device of claim 1 wherein said backing (1300, 2400, 2500, 2900, 3000, 1720) comprises a triangular shape.

11. The tissue approximation device of claim 10 wherein said attachment points (1302, 1600, 2402, 2602, 2902, 3002, 1640, 1670, 1730, 1850) extend from said back side and said anchoring region is disposed on said front side.

12. The tissue approximation device of any one of claims 1, 10 and 11, wherein said device comprises a biodegradable material.

13. The tissue approximation device of claim 12 wherein said biodegradable material comprises a polymer or copolymer.

14. The tissue approximation device of claim 13 wherein said polymer or copolymer comprises one or more materials selected from the group consisting of polyglycolide, polylactide, poly-α-caprolactone, polyglyconate, polylactide-co-glycolide, their mixtures, alloys, and random and block copolymers.

15. The tissue approximation device of any one of claims 1 and 10 to 14 wherein said backing is configured to be flexible.

16. The tissue approximation device of any one of claims 1 and 10 to 15 wherein said backing defines a through-hole (1314, 2510, 2608, 2906, 3006).

17. The tissue approximation device of claim 16 wherein said through-hole (1314, 2510, 2608, 2906, 3006) is slotted.

18. The tissue approximation device of any one of claims 1 and 10 to 17 wherein said attachment points (1302, 1600, 2402, 2602, 2902, 3002, 1640, 1670, 1730, 1850) are varied in density on said backing (1300, 1806, 2104, 2200, 2400, 2500, 2600, 2900, 3000, 1620, 1660, 1720).

19. The tissue approximation device of any one of claims 1 and 10 to 17 wherein said attachment points (1302, 1600, 2402, 2602, 2902, 3002, 1640, 1670, 1730, 1850) are varied in length on said backing (1300, 1806, 2104, 2200, 2400, 2500, 2600, 2900, 3000, 1620, 1660, 1720).

20. The tissue approximation device of any one of claims 1 and 10 to 17 wherein said attachment points (1600) each define a facet (1606) configured to be perpendicular to a plane defined by tissue to be pierced by said attachment points.

21. The tissue approximation device of claim 1 or claim 10 wherein said attachment points (1302, 1600, 2402, 2602, 2902, 3002, 1640, 1670, 1730, 1850) extend from the back side of said backing (1300, 1806, 2104, 2200, 2400, 2500, 2600, 2900, 3000, 1620, 1660, 1720) and said post (1304, 1630, 1690, 1740, 1800, 2000, 2100, 2202, 2204, 2208, 22 10, 2406, 2504, 2604, 2700, 2800, 2904, 3004) extends from the front side of said backing.

22. The tissue approximation device of any one of claims 1 and 10 to 21 wherein said post (1304, 1630, 1690, 1740, 1800, 2000, 2100, 2202, 2204, 2208, 2210, 2406, 2504, 2604, 2700, 2800, 2904, 3004) comprises a distal end and a proximal end, said proximal end being integrally attached to said backing (1300, 1806, 2104, 2200, 2400, 2500, 2600, 2900, 3000, 1620, 1660, 1720).

23. The tissue approximation device of claim 22 wherein said distal end is chamfered (1318).

24. The tissue approximation device of claim 22 or claim 23 wherein said post (1304, 1630, 1690, 1740, 1800, 2000, 2100, 2202, 2204, 2208, 2210, 2406, 2504, 2604, 2700, 2800, 2904, 3004) defines at least one cavity (1306, 1308, 1900, 2408, 2506, 2508).

25. The tissue approximation device of claim 24 wherein said cavity (1900) is defined along a central axis defined by said post (1800).

26. The tissue approximation device of claim 24 wherein said cavity (1306, 1900, 2408, 2506) is defined in said distal end and a second cavity (1308, 2508) is defined in said proximal end.

27. The tissue approximation device of claim 24 or claim 25 wherein said post further defines a through-hole extending axially through said post.

28. The tissue approximation device of any one of claims 1 and 10 to 27 wherein said post (1304, 1630, 1690, 1740, 1800, 2000, 2100, 2202, 2204, 2208, 2210, 2406, 2504, 2604, 2700, 2800, 2904, 3004) further comprises an integral locking device (1310, 1312, 1702, 1704, 1706, 1802, 1804, 2008, 2102, 2208, 2606, 2702, 2802).

29. The tissue approximation device of claim 28 wherein said integral locking device (1310, 1312, 1702, 1704, 1706, 1802, 1804, 2102, 2208, 2606, 2702, 2802) is selected from the group consisting of collars, partial collars, tabs, barbs, subcortical wings and any combinations thereof.

30. The tissue approximation device of any one of claims 1 and 10 to 27 wherein said post is threaded.

31. The tissue approximation device of claim 1 wherein said post comprises a hook.

32. The tissue approximation device of claim 1 wherein said post (1304, 1630, 1690, 1740, 1800, 2000, 2100, 2202, 2204, 2208, 2210, 2406, 2504, 2604, 2700, 2800, 2904, 3004) extends from said backing (1300, 1806, 2104, 2200, 2400, 2500, 2600, 2900, 3000, 1620, 1660, 1720) at a predetermined angle.

33. The tissue approximation device of claim 1 wherein said post (2208) comprises a distal end configured to expand radially about an axis defined by said post.

34. The tissue approximation device of claim 1 wherein said post (2000, 2100) further comprises a latch (2002, 2102) pivotally disposed between post members (2006, 2106, 2102).

35. The tissue approximation device of claim 34 wherein said latch (2002, 2102) is biased to extend angularly away from said post members (2006, 2106).

36. The tissue approximation device of claim 1 further comprising at least one protrusion (1316) integral with said backing (1300).

37. The tissue approximation device of claim 1 wherein said post (1304, 1630, 1690, 1740, 1800, 2000, 2100, 2202, 2204, 2208, 2210, 2406, 2504, 2604, 2700, 2800, 2904, 3004) is configured to be received by a hole (2304, 2306, 2308) defined in a cranium.

38. The tissue approximation device of claim 37 wherein said hole (2306) is angularly defined in said cranium.

39. The tissue approximation device of claim 37 wherein said hole (2308) is defined to have a plurality of concentric grooves.

40. The tissue approximation device of claim 1 wherein said anchoring region defines at least two posts (2904, 3004) extending from said backing (2900, 3000).

41. The tissue approximation device of claim 40 wherein said posts (2904) are adjacent to one another.

42. The tissue approximation device of claim 40 wherein said posts (3004) are disposed along a symmetrical axis defined by said backing (3000).

43. The tissue approximation device of claim 1, wherein said post (1740) is connected to said supportive backing (1720) via an extension member (1710).

44. The tissue approximation device of claim 43 wherein said extension member (1710) is thread-like.

## Patentansprüche

1. Implantierbare Gewebeannäherungsvorrichtung, umfassend:
(a) eine tragende Unterlage;
(b) eine Vielzahl von Befestigungspunkten, die sich von der Unterlage erstrecken; und
(c) eine Knochenverankerungsregion, die einen festen Bestandteil mit der Unterlage bildet, wobei:
die tragende Unterlage (1300, 1806, 2104, 2200, 2400, 2500, 2600, 2900, 3000, 1620, 1660, 1720) einander gegenüber liegende Vorder- und Rückseiten aufweist;
die Vielzahl an Befestigungspunkten (1302, 1600, 2402, 2602, 2902, 3002, 1640, 1670, 1730, 1850) sich jeweils schräg von der Unterlage weg erstrecken; und
die Knochenverankerungsregion einen Bolzen definiert (1304, 1630, 1690, 1740, 1800, 2000, 2100, 2202, 2204, 2208, 2210, 2406, 2504, 2604, 2700, 2800, 2904, 3004), die sich von der Unterlage erstreckt und so konstruiert und angeordnet ist, um die Vorrichtung an dem Knochen festzumachen.

2. Gewebeannäherungsvorrichtung gemäß Anspruch 1, wobei die tragende Unterlage (1300, 1806, 2104, 2200, 2400, 2500, 2600, 2900, 3000, 1620, 1660, 1720) mindestens einen einzelnen Kontaktbereich umfasst und die besagte Vielzahl an Befestigungspunkten (1302, 1600, 2402, 2602, 2902, 3002, 1640, 1670, 1730, 1850) in dem mindestens einen einzelnen Kontaktbereich liegen.

3. Gewebeannäherungsvorrichtung gemäß Anspruch 1, wobei die Befestigungspunkte (1302, 1600, 2402, 2602, 2902, 3002, 1640, 1670, 1730, 1850) mehr als eine Gestalt aufweisen.

4. Gewebeannäherungsvorrichtung gemäß Anspruch 1, wobei die Vorrichtung auch ein Zentrum umfasst und die Befestigungspunkte (1302, 1600, 2402, 2602, 2902, 3002, 1640, 1670, 1730, 1850) zunehmend kürzer werden, je näher sie sich am Zentrum der Vorrichtung befinden.

5. Gewebeannäherungsvorrichtung gemäß Anspruch 1, wobei die Befestigungspunkte (1302, 1600, 2402, 2602, 2902, 3002, 1640, 1670, 1730, 1850) in ihrer Ausrichtung auf der Unterlagen variieren (1300, 1806, 2104, 2200, 2400, 2500, 2600, 2900, 3000, 1620, 1660, 1720).

6. Gewebeannäherungsvorrichtung gemäß Anspruch 1, wobei die Unterlage (1300, 1806, 2104, 2200, 2400, 2500, 2600, 2900, 3000, 1620, 1660, 1720) eine Vielzahl von Öffnungen (2502) umfasst, durch welche Befestigungspunkte (400) gesetzt werden.

7. Gewebeannäherungsvorrichtung gemäß Anspruch 6, wobei Befestigungspunkte (400) an der Unterlage (2500) durch einen Flansch (404) oder eine Muffe festgemacht sind.

8. Gewebeannäherungsvorrichtung gemäß Anspruch 1, welche einen ersten Endbereich mit sich erstreckenden Befestigungspunkten (1302, 1600, 2402, 2602, 2902, 3002, 1640, 1670, 1730, 1850) und einen gegenüber liegenden zweiten Endbereich mit einer oder mehreren Öffnungen durch die Unterlage hindurch umfasst.

9. Gewebeannäherungsvorrichtung von Anspruch 8, wobei die Befestigungspunkte des ersten Endbereichs zu dem zweiten Endbereich hin geneigt sind.

10. Gewebeannäherungsvorrichtung gemäß Anspruch 1, wobei die Unterlage (1300, 2400, 2500, 2900, 3000, 1720) eine dreieckige Gestalt umfasst.

11. Gewebeannäherungsvorrichtung von Anspruch 10, wobei die Befestigungspunkte (1302, 1600, 2402, 2602, 2902, 3002, 1640, 1670, 1730, 1850) sich von der Rückseite erstrecken und die Verankerungsregion auf der Vorderseite angeordnet ist.

12. Gewebeannäherungsvorrichtung gemäß einem Beliebigen der Ansprüche 1, 10 und 11, wobei die Vorrichtung ein biologisch abbaubares Material umfasst.

13. Gewebeannäherungsvorrichtung gemäß Anspruch 12, wobei das biologisch abbaubare Material ein Polymer oder Copolymer umfasst.

14. Gewebeannäherungsvorrichtung von Anspruch 13, wobei das Polymer oder Copolymer ein oder mehrere Materialien umfasst, die aus der Gruppe, bestehend aus Polyglycolid, Polylactid, Poly-α-caprolacton, Polyglyconat, Polylactid-co-glycolid, deren Mischungen, Legierungen und statistischen und Blockcopolymeren, gewählt werden.

15. Gewebeannäherungsvorrichtung gemäß einem Beliebigen der Ansprüche 1 und 10 bis 14, wobei die Unterlage so gestaltet ist, dass sie flexibel ist.

16. Gewebeannäherungsvorrichtung gemäß einem Beliebigen der Ansprüche 1 und 10 bis 15, wobei die Unterlage ein Durchgangsloch (1314, 2510, 2608, 2906, 3006) definiert.

17. Gewebeannäherungsvorrichtung von Anspruch 16, wobei das Durchgangsloch (1314, 2510, 2608, 2906, 3006) geschlitzt ist.

18. Gewebeannäherungsvorrichtung gemäß einem Beliebigen der Ansprüche 1 und 10 bis 17, wobei die Befestigungspunkte (1302, 1600, 2402, 2602, 2902, 3002, 1640, 1670, 1730, 1850) eine unterschiedliche Dichte auf der Unterlage haben (1300, 1806, 2104, 2200, 2400, 2500, 2600, 2900, 3000, 1620, 1660, 1720).

19. Gewebeannäherungsvorrichtung gemäß einem Beliebigen der Ansprüche 1 und 10 bis 17, wobei die Befestigungspunkte (1302, 1600, 2402, 2602, 2902, 3002, 1640, 1670, 1730, 1850) eine unterschiedliche Länge auf der Unterlage haben (1300, 1806, 2104, 2200, 2400, 2500, 2600, 2900, 3000, 1620, 1660, 1720).

20. Gewebeannäherungsvorrichtung gemäß einem Beliebigen der Ansprüche 1 und 10 bis 17, wobei die Befestigungspunkte (1600) jeweils eine Facette (1606) bilden, die senkrecht zu einer durch Gewebe definierten Ebene konfiguriert ist, das durch die Befestigungspunkte zu durchbohren ist.

21. Gewebeannäherungsvorrichtung gemäß Anspruch 1 oder Anspruch 10, wobei die Befestigungspunkte (1302, 1600, 2402, 2602, 2902, 3002, 1640, 1670, 1730, 1850) sich von der Rückseite der Unterlage (1300, 1806, 2104, 2200, 2400, 2500, 2600, 2900, 3000, 1620, 1660, 1720) erstrecken und der Bolzen (1304, 1630, 1690, 1740, 1800, 2000, 2100, 2202, 2204, 2208, 2210, 2406, 2504, 2604, 2700, 2800, 2904, 3004) sich von der Vorderseite der Unterlage erstreckt.

22. Gewebeannäherungsvorrichtung gemäß einem Beliebigen der Ansprüche 1 und 10 bis 21, wobei der Bolzen (1304, 1630, 1690, 1740, 1800, 2000, 2100, 2202, 2204, 2208, 2210, 2406, 2504, 2604, 2700, 2800, 2904, 3004) ein distales Ende und ein proximales Ende umfasst, wobei das proximale Ende an der Unterlage (1300, 1806, 2104, 2200, 2400, 2500, 2600, 2900, 3000, 1620, 1660, 1720) in integrierter Weise befestigt ist.

23. Gewebeannäherungsvorrichtung gemäß Anspruch 22, wobei das distale Ende abgeschrägt ist (1318).

24. Gewebeannäherungsvorrichtung gemäß Anspruch 22 oder Anspruch 23, wobei der Bolzen (1304, 1630, 1690, 1740, 1800, 2000, 2100, 2202, 2204, 2208, 2210, 2406, 2504, 2604, 2700, 2800, 2904, 3004) mindestens einen Hohlraum bildet (1306, 1308, 1900, 2408, 2506, 2508).

25. Gewebeannäherungsvorrichtung gemäß Anspruch 24, wobei der Hohlraum (1900) entlang einer durch den Bolzen (1800) gebildeten zentralen Achse definiert ist.

26. Gewebeannäherungsvorrichtung gemäß Anspruch 24, wobei der Hohlraum (1306, 1900, 2408, 2506) in dem distalen Ende definiert ist und ein sekundärer Hohlraum (1308, 2508) in dem proximalen Ende definiert ist.

27. Gewebeannäherungsvorrichtung gemäß Anspruch 24 oder Anspruch 25, wobei der Bolzen weiter ein Durchgangloch definiert, das sich axial durch den Bolzen erstreckt.

28. Gewebeannäherungsvorrichtung gemäß einem Beliebigen der Ansprüche 1 und 10 bis 27, wobei der Bolzen (1304, 1630, 1690, 1740, 1800, 2000, 2100, 2202, 2204, 2208, 2210, 2406, 2504, 2604, 2700, 2800, 2904, 3004) weiter eine integrale Arretierungseinrichtung umfasst (1310, 1312, 1702, 1704, 1706, 1802, 1804, 2008, 2102, 2208, 2606, 2702, 2802).

29. Gewebeannäherungsvorrichtung gemäß Anspruch 28, wobei die integrale Arretierungseinrichtung (1310, 1312, 1702, 1704, 1706, 1802, 1804, 2102, 2208, 2606, 2702, 2802) aus der Gruppe gewählt ist, die aus Bügeln, teilweisen Bügeln, Laschen, Widerhaken, subkortikalen Flügeln und beliebigen Kombinationen davon besteht.

30. Gewebeannäherungsvorrichtung gemäß einem Beliebigen der Ansprüche 1 und 10 bis 27, wobei der Bolzen gewindeförmig ist.

31. Gewebeannäherungsvorrichtung gemäß Anspruch 1, wobei der Bolzen einen Haken umfasst.

32. Gewebeannäherungsvorrichtung gemäß Anspruch 1, wobei der Bolzen (1304, 1630, 1690, 1740, 1800, 2000, 2100, 2202, 2204, 2208, 2210, 2406, 2504, 2604, 2700, 2800, 2904, 3004) sich von der Unterlage (1300, 1806, 2104, 2200, 2400, 2500, 2600, 2900, 3000, 1620, 1660, 1720) in einem vorbestimmten Winkel erstreckt.

33. Gewebeannäherungsvorrichtung gemäß Anspruch 1, wobei der Bolzen (2208) ein distales Ende umfasst, das so ausgelegt ist, dass es sich radial um eine durch den Bolzen definierte Achse erstreckt.

34. Gewebeannäherungsvorrichtung gemäß Anspruch 1, wobei der Bolzen (2000, 2100) weiter einen Riegel (2002, 2102) umfasst, der zwischen Bolzenteilen (2006, 2106, 2102) drehgelenkig angeordnet ist.

35. Gewebeannäherungsvorrichtung gemäß Anspruch 34, wobei der Riegel (2002, 2102) geneigt ist, um sich schräg von den Bolzenteilen zu erstrecken (2006, 2106).

36. Gewebeannäherungsvorrichtung gemäß Anspruch 1, weiterhin umfassend mindestens eine Erhebung (1316), die mit der Unterlage (1300) einen festen Bestandteil bildet.

37. Gewebeannäherungsvorrichtung gemäß Anspruch 1, wobei der Bolzen (1304, 1630, 1690, 1740, 1800, 2000, 2100, 2202, 2204, 2208, 2210, 2406, 2504, 2604, 2700, 2800, 2904, 3004) so ausgelegt ist, um von einem in einem Schädel definierten Loch (2304, 2306, 2308) aufgenommen zu werden.

38. Gewebeannäherungsvorrichtung gemäß Anspruch 37, wobei das Loch (2306) abgewinkelt in dem Schädel definiert ist.

39. Gewebeannäherungsvorrichtung gemäß Anspruch 37, wobei das Loch (2308) so definiert ist, dass es eine Vielzahl an konzentrischen Rillen aufweist.

40. Gewebeannäherungsvorrichtung gemäß Anspruch 1, wobei die Verankerungsregion mindestens zwei Bolzen (2904, 3004) definiert, die sich von der Unterlage (2900, 3000) erstrecken.

41. Gewebeannäherungsvorrichtung gemäß Anspruch 40, wobei die Bolzen (2904) einander benachbart sind.

42. Gewebeannäherungsvorrichtung gemäß Anspruch 40, wobei die Bolzen (3004) entlang einer durch die Unterlage (3000) definierten symmetrischen Achse angeordnet sind.

43. Gewebeannäherungsvorrichtung gemäß Anspruch 1, wobei der Bolzen (1740) mit der tragenden Unterlage (1720) mittels eines Erweiterungsteils (1710) verbunden ist.

44. Gewebeannäherungsvorrichtung gemäß Anspruch 43, wobei das Erweiterungsteil (1710) gewindeförmig ist.

## Revendications

1. Dispositif d'affrontement de tissus implantable comprenant :
(a) un support;
(b) une pluralité de points de fixation s'étendant depuis ledit support ; et
(c) une région d'ancrage osseux intégrée audit support, dans laquelle :
le support (1300, 1806, 2104, 2200, 2400, 2500, 2600, 2900, 3000, 1620, 1660, 1720) a des côtés avant et arrière opposés ;
les points de fixation (1302, 1600, 2402, 2602, 2902, 3002, 1640, 1670, 1730, 1850) s'étendent tous depuis ledit support de manière inclinée ; et
ladite région d'ancrage osseux définit un montant (1304, 1630, 1690, 1740, 1800, 2000, 2100, 2202, 2204, 2208, 2210, 2406, 2504, 2604, 2700, 2800, 2904, 3004) s'étendant depuis ledit support et construit et agencé pour assujettir le dispositif à un os.

2. Dispositif d'affrontement de tissus selon la revendication 1, dans lequel ledit support (1300, 1806, 2104, 2200, 2400, 2500, 2600, 2900, 3000, 1620, 1660, 1720) comprend au moins une zone de contact discrète et lesdits points de fixation (1302, 1600, 2402, 2602, 2902, 3002, 1640, 1670, 1730, 1850) sont situés dans ladite au moins une zone de contact discrète.

3. Dispositif d'affrontement de tissus selon la revendication 1, dans lequel lesdits points de fixation (1302, 1600, 2402, 2602, 2902, 3002, 1640, 1670, 1730, 1850) ont plus d'une forme.

4. Dispositif d'affrontement de tissus selon la revendication 1, dans lequel ledit dispositif comprend aussi un centre et lesdits points de fixation (1302, 1600, 2402, 2602, 2902, 3002, 1640, 1670, 1730, 1850) sont progressivement plus courts en se rapprochant du centre dudit dispositif.

5. Dispositif d'affrontement de tissus selon la revendication 1, dans lequel lesdits points de fixation (1302, 1600, 2402, 2602, 2902, 3002, 1640, 1670, 1730, 1850) ont une orientation qui varie sur ledit support (1300, 1806, 2104, 2200, 2400, 2500, 2600, 2900, 3000, 1620, 1660, 1720).

6. Dispositif d'affrontement de tissus selon la revendication 1, dans lequel ledit support (1300, 1806, 2104, 2200, 2400, 2500, 2600, 2900, 3000, 1620, 1660, 1720) comprend une pluralité d'ouvertures (2502) à travers lesquelles sont placés des points de fixation (400).

7. Dispositif d'affrontement de tissus selon la revendication 6, dans lequel lesdits points de fixation (400) sont fixés au support (2500) par une bride (404) ou une joue.

8. Dispositif d'affrontement de tissus selon la revendication 1, comprenant une première zone d'extrémité comportant des points de fixation étendus (1302, 1600, 2402, 2602, 2902, 3002, 1640, 1670, 1730, 1850) et une deuxième zone d'extrémité opposée comportant une ou plusieurs ouvertures dans le support.

9. Dispositif d'affrontement de tissus selon la revendication 8, dans lequel les points de fixation de ladite première zone d'extrémité sont inclinés vers ladite deuxième zone d'extrémité.

10. Dispositif d'affrontement de tissus selon la revendication 1, dans lequel ledit support (1300, 2400, 2500, 2900, 3000, 1720) comprend une forme triangulaire.

11. Dispositif d'affrontement de tissus selon la revendication 10, dans lequel lesdits points de fixation (1302, 1600, 2402, 2602, 2902, 3002, 1640, 1670, 1730, 1850) s'étendent depuis ledit côté arrière et ladite région d'ancrage est disposée sur ledit côté avant.

12. Dispositif d'affrontement de tissus selon l'une quelconque des revendications 1, 10 et 11, dans lequel ledit dispositif comprend un matériau biodégradable.

13. Dispositif d'affrontement de tissus selon la revendication 12, dans lequel ledit matériau biodégradable comprend un polymère ou un copolymère.

14. Dispositif d'affrontement de tissus selon la revendication 13, dans lequel ledit polymère ou copolymère comprend un ou plusieurs matériau(x) choisi(s) dans l'ensemble constitué par les polyglycolides, polylactides, poly-α-caprolactone, polyglyconates et poly(lactide-co-glycolide), ainsi que leurs mélanges, alliages, et copolymères statistiques ou à blocs.

15. Dispositif d'affrontement de tissus selon l'une quelconque des revendications 1 et 10 à 14, dans lequel ledit support est configuré pour être flexible.

16. Dispositif d'affrontement de tissus selon l'une quelconque des revendications 1 et 10 à 15, dans lequel ledit support définit un trou traversant (1314, 2510, 2608, 2906, 3006).

17. Dispositif d'affrontement de tissus selon la revendication 16, dans lequel ledit trou traversant (1314, 2510, 2608, 2906, 3006) est rainuré.

18. Dispositif d'affrontement de tissus selon l'une quelconque des revendications 1 et 10 à 17, dans lequel lesdits points de fixation (1302, 1600, 2402, 2602, 2902, 3002, 1640, 1670, 1730, 1850) varient en densité sur ledit support (1300, 1806, 2104, 2200, 2400, 2500, 2600, 2900, 3000, 1620, 1660, 1720).

19. Dispositif d'affrontement de tissus selon l'une quelconque des revendications 1 et 10 à 17, dans lequel lesdits points de fixation (1302, 1600, 2402, 2602, 2902, 3002, 1640, 1670, 1730, 1850) varient en longueur sur ledit support (1300, 1806, 2104, 2200, 2400, 2500, 2600, 2900, 3000, 1620, 1660, 1720).

20. Dispositif d'affrontement de tissus selon l'une quelconque des revendications 1 et 10 à 17, dans lequel lesdits points de fixation (1600) définissent chacun une facette (1606) configurée pour être perpendiculaire à un plan défini par le tissu devant être percé par lesdits points de fixation.

21. Dispositif d'affrontement de tissus selon la revendication 1 ou 10, dans lequel lesdits points de fixation (1302, 1600, 2402, 2602, 2902, 3002, 1640, 1670, 1730, 1850) s'étendent depuis le côté arrière dudit support (1300, 1806, 2104, 2200, 2400, 2500, 2600, 2900, 3000, 1620, 1660, 1720) et ledit montant (1304, 1630, 1690, 1740, 1800, 2000, 2100, 2202, 2204, 2208, 2210, 2406, 2504, 2604, 2700, 2800, 2904, 3004) s'étend depuis le côté avant dudit support.

22. Dispositif d'affrontement de tissus selon l'une quelconque des revendications 1 et 10 à 21, dans lequel ledit montant (1304, 1630, 1690, 1740, 1800, 2000, 2100, 2202, 2204, 2208, 2210, 2406, 2504, 2604, 2700, 2800, 2904, 3004) comprend une extrémité distale et une extrémité proximale, ladite extrémité proximale étant fixée d'un seul tenant audit support (1300, 1806, 2104, 2200, 2400, 2500, 2600, 2900, 3000, 1620, 1660, 1720).

23. Dispositif d'affrontement de tissus selon la revendication 22, dans lequel ladite extrémité distale est chanfreinée (1318).

24. Dispositif d'affrontement de tissus selon la revendication 22 ou 23, dans lequel ledit montant (1304, 1630, 1690, 1740, 1800, 2000, 2100, 2202, 2204, 2208, 2210, 2406, 2504, 2604, 2700, 2800, 2904, 3004) définit au moins une cavité (1306, 1308, 1900, 2408, 2506, 2508).

25. Dispositif d'affrontement de tissus selon la revendication 24, dans lequel ladite cavité (1900) est définie le long d'un axe central défini par ledit montant (1800).

26. Dispositif d'affrontement de tissus selon la revendication 24, dans lequel ladite cavité (1306, 1900, 2408, 2506) est définie dans ladite extrémité distale et une seconde cavité (1308, 2508) est définie dans ladite extrémité proximale.

27. Dispositif d'affrontement de tissus selon la revendication 24 ou 25, dans lequel ledit montant définit en outre un trou traversant s'étendant axialement dans ledit montant.

28. Dispositif d'affrontement de tissus selon l'une quelconque des revendications 1 et 10 à 27, dans lequel ledit montant (1304, 1630, 1690, 1740, 1800, 2000, 2100, 2202, 2204, 2208, 2210, 2406, 2504, 2604, 2700, 2800, 2904, 3004) comprend en outre un dispositif de blocage intégré (1310, 1312, 1702, 1704, 1706, 1802, 1804, 2008, 2102, 2208, 2606, 2702, 2802).

29. Dispositif d'affrontement de tissus selon la revendication 28, dans lequel ledit dispositif de blocage intégré (1310, 1312, 1702, 1704, 1706, 1802, 1804, 2008, 2102, 2208, 2606, 2702, 2802) est choisi dans l'ensemble comprenant les colliers, les colliers partiels, les pattes, les crans, les ailes sous-corticales et toutes leurs combinaisons.

30. Dispositif d'affrontement de tissus selon l'une quelconque des revendications 1 et 10 à 27, dans lequel ledit montant est fileté.

31. Dispositif d'affrontement de tissus selon la revendication 1, dans lequel ledit montant comprend un crochet.

32. Dispositif d'affrontement de tissus selon la revendication 1, dans lequel ledit montant (1304, 1630, 1690, 1740, 1800, 2000, 2100, 2202, 2204, 2208, 2210, 2406, 2504, 2604, 2700, 2800, 2904, 3004) s'étend depuis ledit support (1300, 1806, 2104, 2200, 2400, 2500, 2600, 2900, 3000, 1620, 1660, 1720) en formant un angle prédéterminé.

33. Dispositif d'affrontement de tissus selon la revendication 1, dans lequel ledit montant (2208) comprend une extrémité distale configurée pour se dilater radialement autour d'un axe défini par ledit montant.

34. Dispositif d'affrontement de tissus selon la revendication 1, dans lequel ledit montant (2000, 2100) comprend en outre un loquet (2002, 2102) placé de façon pivotante entre des éléments de montant (2006, 2106, 2102).

35. Dispositif d'affrontement de tissus selon la revendication 34, dans lequel ledit loquet (2002, 2102) est sollicité pour s'étendre angulairement à l'écart desdits éléments de montant (2006, 2106).

36. Dispositif d'affrontement de tissus selon la revendication 1, comprenant en outre au moins une protubérance (1316) intégrée audit support (1300).

37. Dispositif d'affrontement de tissus selon la revendication 1, dans lequel ledit montant (1304, 1630, 1690, 1740, 1800, 2000, 2100, 2202, 2204, 2208, 2210, 2406, 2504, 2604, 2700, 2800, 2904, 3004) est configuré pour être reçu par un trou (2304, 2306, 2308) défini dans une boîte crânienne.

38. Dispositif d'affrontement de tissus selon la revendication 37, dans lequel ledit trou (2306) est défini de façon angulaire dans ladite boîte crânienne.

39. Dispositif d'affrontement de tissus selon la revendication 37, dans lequel ledit trou (2308) est défini de façon à avoir une pluralité de rainures concentriques.

40. Dispositif d'affrontement de tissus selon la revendication 1, dans lequel ladite région d'ancrage définit au moins deux montants (2904, 3004) s'étendant depuis ledit support (2900, 3000).

41. Dispositif d'affrontement de tissus selon la revendication 40, dans lequel lesdits montants (2904) sont adjacents entre eux.

42. Dispositif d'affrontement de tissus selon la revendication 40, dans lequel lesdits montants (3004) sont disposés le long d'un axe de symétrie défini par ledit support (3000).

43. Dispositif d'affrontement de tissus selon la revendication 1, dans lequel ledit montant (1740) est relié audit support (1720) par un élément d'extension (1710).

44. Dispositif d'affrontement de tissus selon la revendication 43, dans lequel ledit élément d'extension (1710) est en forme de fil.
